(19) Europäisches Patentamt
European Patent Office
Office européen des brevets

(11) **EP 1 933 729 B1**

(12) **FASCICULE DE BREVET EUROPEEN**

(45) Date de publication et mention de la délivrance du brevet:
**19.10.2011 Bulletin 2011/42**

(21) Numéro de dépôt: **06778412.4**

(22) Date de dépôt: **30.08.2006**

(51) Int Cl.:
*A61B 17/22* (2006.01)    *A61B 5/055* (2006.01)

(86) Numéro de dépôt international:
**PCT/EP2006/065832**

(87) Numéro de publication internationale:
**WO 2007/036409 (05.04.2007 Gazette 2007/14)**

(54) **DISPOSITIF DE TRAITEMENT THERMIQUE DE TISSUS BIOLOGIQUES EN MOUVEMENT, ET PROCEDE DE POSITIONNEMENT ASSOCIE**

VORRICHTUNG ZUR WÄRMEBEHANDLUNG VON BEWEGLICHEM BIOLOGISCHEM GEWEBE UND RELEVANTES POSITIONIERUNG VERFAHREN

DEVICE FOR HEAT TREATING MOVING BIOLOGICAL TISSUES, AND RELATED POSITIONING METHOD

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **28.09.2005 FR 0509885**

(43) Date de publication de la demande:
**25.06.2008 Bulletin 2008/26**

(73) Titulaires:
• **Centre National de la Recherche Scientifique 75016 Paris (FR)**
• **Image Guided Therapy 33600 Pessac (FR)**

(72) Inventeurs:
• **MOONEN, Chrétien 33170 Gradignan (FR)**
• **MOUGENOT, Charles 33000 Bordeaux (FR)**
• **DENIS DE SENNEVILLE, Baudouin 33200 Bordeaux (FR)**

(74) Mandataire: **Tetaz, Franck Claude Edouard et al Cabinet Regimbeau 139, rue Vendôme 69477 Lyon Cedex 06 (FR)**

(56) Documents cités:
EP-A- 1 110 508      WO-A-97/22015
WO-A-2004/075987     US-A- 5 178 135
US-A1- 2003 088 174  US-A1- 2004 019 447

**Description**

DOMAINE DE L'INVENTION

L'invention concerne le domaine du traitement de tissus biologiques par hyperthermie.

ETAT DE LA TECHNIQUE

Les thérapies par hyperthermie sont des techniques couramment utilisées pour traiter localement des tissus biologiques. Elles consistent à chauffer au moyen d'une source d'énergie (laser, micro-ondes, ondes radiofréquences, ultrasons) une région cible du tissu biologique.

D'une manière générale, les thérapies par hyperthermie locale permettent des interventions médicales dont la nature invasive est réduite au minimum. Parmi les différents types d'énergie utilisés, les ultrasons focalisés (FUS) sont particulièrement intéressants puisqu'ils permettent de chauffer une région cible, de manière non invasive et en profondeur dans les tissus.

Pendant le traitement, la température de la zone cible et de son environnement immédiat doit être contrôlée de manière précise et continue. Pour que le dispositif de traitement soit totalement non invasif, il sera par exemple possible d'utiliser l'Imagerie par Résonance Magnétique (IRM) qui permet d'obtenir une cartographie précise des distributions de températures ainsi que des informations anatomiques détaillées.

Les systèmes non invasifs ont néanmoins souvent une position fixe alors que les tissus biologiques, et par conséquent les régions cibles à traiter, sont animés de mouvements de toutes natures.

Le document EP 1 110 508 porte sur un dispositif de traitement médical d'un tissu par ultrasons, dans lequel le positionnement du point focal de traitement est déterminé à partir d'une image de la région à traiter, cette image étant fournie par un dispositif de tomographie.

Le document US 5,938,600 présente un procédé de traitement d'un tissu biologique en mouvement et un dispositif associé. Il est prévu selon ce procédé de déterminer automatiquement le mouvement de la région cible en mouvement par un système IRM puis de générer un signal représentant le mouvement ainsi déterminé, ce signal permettant à un dispositif ultrasons d'émettre un rayonnement dans une région focale suivant la région cible en mouvement. Néanmoins, un tel procédé de traitement ne permet qu'une correction partielle du mouvement de la région cible et certaines zones du tissu biologique peuvent être encore indûment irradiées.

Un but de la présente invention est donc de fournir un dispositif amélioré de traitement de tissus biologiques en mouvement, ce dispositif permettant de résoudre au moins l'un des inconvénients précités.

EXPOSE DE L'INVENTION

On prévoit à cet effet selon l'invention un dispositif de traitement thermique d'une région cible en mouvement d'un tissu biologique, comprenant des moyens de calcul pour estimer une position de la région cible à partir d'un signal de mesure de la région cible, caractérisé en ce qu'il comprend en outre des moyens de commande pour positionner un point de traitement dans la région cible en fonction de la position estimée et d'un délai de positionnement écoulé entre une mesure du signal de mesure de la région cible et le positionnement du point de traitement, de façon à compenser le mouvement de la région cible pendant le délai de positionnement.

Des aspects préférés mais non limitatifs du dispositif de traitement thermique selon l'invention sont les suivants :

- le délai de positionnement comprend un délai de latence dû à l'estimation de la position de la région cible par les moyens de calcul, de sorte que les moyens de commande sont aptes à compenser le mouvement de la région cible pendant le délai de latence ;
- le délai de positionnement comprend un délai d'anticipation du mouvement de la région cible, de sorte que les moyens de commande sont aptes à anticiper et compenser le mouvement de la région cible pendant le temps d'anticipation ;
- le dispositif de traitement comprend en outre des moyens de mesure du délai de latence à transmettre aux moyens de commande ;
- le dispositif de traitement comprend en outre des moyens de modélisation pour modéliser le mouvement de la région cible à partir d'une succession de signaux de mesure de la région cible, la modélisation du mouvement pouvant être périodique ;
- les moyens de modélisation sont aptes à fournir une position spatiale de la région cible en fonction d'une position temporelle, les positions spatiale et temporelle définissant la position de la région cible ;
- les moyens de calcul sont aptes à déterminer une position spatiale estimée de la région cible en utilisant un algorithme de traitement du signal de mesure de la région cible ;

- les moyens de calcul sont aptes à déterminer, selon la modélisation du mouvement de la région cible, une position temporelle estimée de la région cible correspondant à la position spatiale estimée ;
- les moyens de commande sont aptes à positionner le point de traitement selon une position spatiale réelle de la région cible, la position spatiale réelle étant fonction d'une position temporelle réelle selon la modélisation du mouvement de la région cible, la position temporelle réelle correspondant à la position temporelle estimée augmentée du délai de latence ;
- les moyens de commande peuvent en outre être aptes à positionner le point de traitement entre des estimations successives par les moyens de calcul d'une première et d'une deuxième position temporelle estimées à partir respectivement d'un premier et d'un deuxième signal de mesure de la région cible, de façon à anticiper le mouvement de la région cible ;
- les moyens de commande sont aptes à positionner le point de traitement selon une position spatiale anticipée, la position spatiale anticipée étant fonction d'une position temporelle anticipée selon la modélisation du mouvement de la région cible, la position temporelle anticipée correspondant à la position temporelle estimée augmentée du délai de latence et du délai d'anticipation.
- les moyens de calcul sont aptes à déterminer un champ de vecteurs de déplacement estimé de la région cible en utilisant un algorithme de traitement du signal de mesure de la région cible ;
- les moyens de commande sont aptes à positionner le point de traitement dans la région cible en fonction du champ de vecteurs de déplacement estimé ;
- le signal de mesure de la région cible est mesuré par des moyens d'imagerie pour fournir une image anatomique de la région cible ;
- les moyens d'imagerie peuvent en outre être aptes à fournir une image de phase de la région cible à partir du signal de mesure de la région cible, pour le suivi des variations de température de la région cible à partir d'une image de phase de référence ;
- les moyens de calcul peuvent en outre être aptes à modifier l'image de phase de référence pour corriger une perturbation de température due à un mouvement de la région cible, en utilisant par exemple un historique d'images de phase ;
- le dispositif de traitement peut en outre comprendre des moyens de régulation d'un rayonnement appliqué sur le point de traitement dans la région cible pour que la distribution spatiale de température de la région cible soit conforme à une consigne de distribution spatiale de température, les moyens de régulation étant aptes à réguler le rayonnement appliqué en fonction de la distribution spatiale de température de la région cible et d'une consigne de distribution spatiale de température, selon une loi de régulation comprenant un terme Proportionnel-Intégral-Dérivé ;
- le traitement thermique de la région cible est non invasif.

On prévoit en outre selon l'invention un procédé de position traitement thermique d'une région cible en mouvement d'un tissu biologique, caractérisé en ce qu'il comprend les étapes consistant à :

- mesurer la région cible pour obtenir un signal de mesure de la région cible,
- estimer une position de la région cible à partir du signal de mesure de la région cible par des moyens de calcul,
- positionner un point de traitement dans la région cible par des moyens de commande, en fonction de la position estimée et d'un délai de positionnement écoulé entre la mesure du signal de mesure de la région cible et le positionnement du point de traitement, de façon à compenser le mouvement de la région cible pendant le délai de positionnement.

Des aspects préférés mais non limitatifs du procédé de traitement thermique selon l'invention sont les suivants :

- le délai de positionnement comprend un délai de latence dû à l'estimation de la position de la région cible par les moyens de calcul, de façon à compenser le mouvement de la région cible pendant le délai de latence ;
- le délai de positionnement comprend un délai d'anticipation du mouvement de la région cible, de façon à anticiper et compenser le mouvement de la région cible pendant le temps d'anticipation ;
- le procédé de traitement comprend en outre l'étape consistant à mesurer en temps réel le délai de latence par des moyens de mesure ;
- le procédé de traitement comprend en outre l'étape consistant à modéliser le mouvement de la région cible à partir d'une succession de signaux de mesure de la région cible, au cours de laquelle on peut déterminer une période type pour la modélisation du mouvement de la région cible ;
- on modélise la position de la région cible par une position spatiale en fonction d'une position temporelle ;
- l'estimation de la position de la région cible comprend les étapes consistant à :

o déterminer une position spatiale estimée de la région cible en utilisant un algorithme de traitement du signal de mesure de la région cible, et

o déterminer, selon la modélisation du mouvement de la région cible, une position temporelle estimée de la région cible correspondant à la position spatiale estimée.

- le positionnement du point de traitement comprend les étapes consistant à :

o déterminer une position temporelle réelle de la région cible, la position temporelle réelle correspondant à la position temporelle estimée augmentée du délai de latence,

o déterminer une position spatiale réelle de la région cible, selon la modélisation du mouvement de la région cible, en fonction de la position temporelle réelle, et

o positionner le point de traitement selon la position spatiale réelle.

- le positionnement du point de traitement comprend les étapes supplémentaires consistant à :

o déterminer une position temporelle anticipée de la région cible, la position temporelle anticipée correspondant à la position temporelle estimée augmentée du délai de latence et d'un délai d'anticipation,

o déterminer une position spatiale anticipée de la région cible, selon la modélisation du mouvement de la région cible, en fonction de la position temporelle anticipée, et

o positionner le point de traitement selon la position spatiale anticipée.

- les étapes supplémentaires du positionnement du point de traitement peuvent être renouvelées jusqu'à ce qu'une nouvelle position temporelle estimée soit déterminée ;
- le procédé de traitement peut en outre comprendre l'étape consistant à déterminer un champ de vecteurs de déplacement estimé de la région cible en utilisant un algorithme de traitement du signal de mesure de la région cible, le positionnement du point de traitement dans la région cible étant fonction du champ de vecteurs de déplacement estimé ;
- le signal de mesure de la région cible fournit une image anatomique de la région cible pour le positionnement du point de traitement, et peut en outre fournir une image de phase de la région cible.
- le procédé de traitement peut en outre comprendre les étapes consistant à :

o déterminer une image de phase de référence ;

o comparer l'image de phase acquise et l'image de phase de référence pour suivre les variations de température de la région cible.

- on détermine une image de phase de référence à partir d'un historique d'images de phase ;
- le procédé de traitement peut en outre comprendre une étape consistant à réguler un rayonnement appliqué sur le point de traitement dans la région cible pour que la distribution spatiale de température de la région cible soit conforme à une consigne de distribution spatiale de température ;
- on régule le rayonnement appliqué en fonction de la distribution spatiale de température de la région cible et d'une consigne de distribution spatiale de température, selon une loi de régulation comprenant un terme Proportionnel-Intégral-Dérivé ;
- le procédé de traitement est réalisé de manière non invasive.

## DESCRIPTION DES FIGURES

D'autres caractéristiques et avantages ressortiront encore de la description qui suit, laquelle est purement illustrative et non limitative et doit être lue en regard des figures annexées parmi lesquelles :

- la figure 1 est une représentation schématique du dispositif de traitement selon l'invention ;
- les figures 2a à 2e illustrent une méthode de recalage d'images ;
- la figure 3 est une représentation schématique de la plateforme d'évaluation du dispositif de traitement selon l'invention ;
- les figures 4a et 4b sont des représentations des mouvements. de la région cible selon une première évaluation de la correction de la position du point de traitement par le dispositif de traitement selon l'invention ;
- les figures 5a et 5b sont des représentations des mouvements de la région cible selon une deuxième évaluation de la correction de la position du point de traitement par le dispositif de traitement selon l'invention ;
- les figures 6a et 6b sont des représentations des mouvements de la région cible selon une troisième évaluation de la correction de la position du point de traitement par le dispositif de traitement selon l'invention ;

- les figures 7a et 7b sont des représentations des mouvements de la région cible selon une quatrième évaluation de la correction de la position du point de traitement par le dispositif de traitement selon l'invention ;
- les figures 8a à 8f illustrent une première évaluation de la correction de la thermométrie et de la position du point de traitement par le dispositif de traitement selon l'invention ;
- la figure 9 est une représentation de la répartition spatiale de température selon la première évaluation illustrée aux figures 8a à 8f ;
- la figure 10 est une représentation de l'évolution temporelle de la température selon la première évaluation illustrée aux figures 8a à 8f ;
- les figures 11a à 11f illustrent une deuxième évaluation de la correction de la thermométrie et de la position du point de traitement par le dispositif de traitement selon l'invention ;
- la figure 12 est une représentation de la répartition spatiale de température selon la deuxième évaluation illustrée aux figures 11a à 11f;
- la figure 13 est une représentation de l'évolution temporelle de la température selon la deuxième évaluation illustrée aux figures 11a à 11f ;
- les figures 14a et 14b représentent les images nécessaires au recalage d'images lors d'un mouvement élastique périodique ;
- les figures 15a à 15f illustrent la troisième évaluation de la correction de la thermométrie et de la position du point de traitement par le dispositif de traitement selon l'invention ;
- la figure 16 est une représentation de la répartition spatiale de température selon la deuxième évaluation illustrée aux figures 15a à 15f ;
- la figure 17 est une représentation de l'évolution temporelle de la température selon la deuxième évaluation illustrée aux figures 15a à 15f ;
- les figures 18a et 18b sont des graphes illustrant une première évaluation de l'asservissement de la température par le dispositif de traitement selon l'invention corrigeant la position du point de traitement ;
- les figures 19a à 19d sont des images IRM illustrant la première évaluation de l'asservissement de la température ;
- les figures 20a à 20d sont des représentations de la répartition spatiale de température selon la première évaluation de l'asservissement de la température ;
- les figures 21 a et 21 b sont des graphes illustrant une deuxième évaluation de l'asservissement de la température par le dispositif de traitement selon l'invention corrigeant la position du point de traitement ;
- les figures 22a à 22d sont des images IRM illustrant la deuxième évaluation de l'asservissement de la température ;
- les figures 23a à 23d sont des représentations de la répartition spatiale de température selon la deuxième évaluation de l'asservissement de la température.

DESCRIPTION DETAILLEE DE L'INVENTION

### Description du dispositif de traitement de tissus biologiques en mouvement selon l'invention et de son fonctionnement

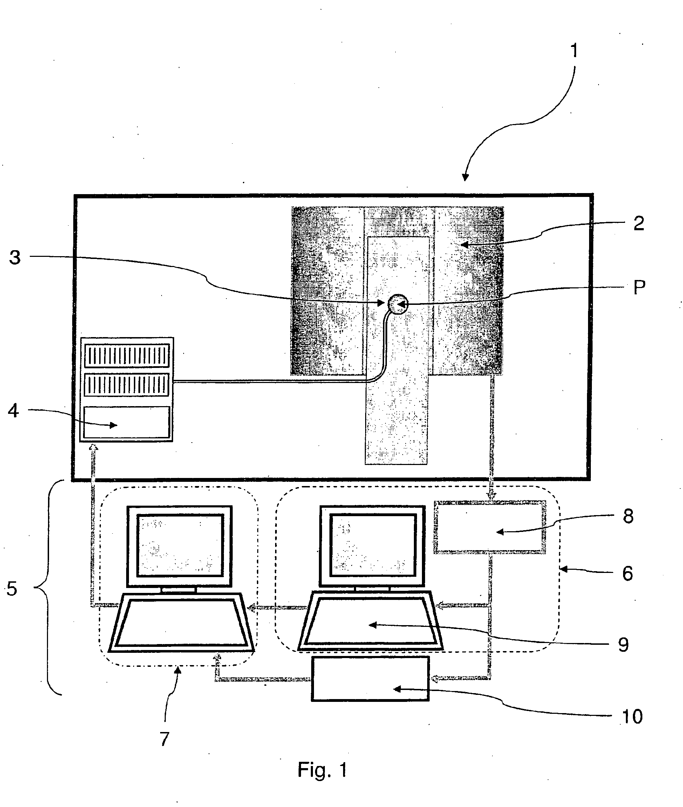

Sur la figure 1 est représenté un dispositif de traitement de tissus biologiques comprenant des moyens de mesure d'une région cible d'un tissu biologique à traiter pour fournir un signal de mesure de la région cible destiné à caractériser la région cible (son mouvement par exemple). Pour un traitement non invasif, on peut par exemple utiliser comme moyens de mesure des moyens d'imagerie IRM 2 destinés à fournir des images de la région cible du tissu biologique à traiter. Cet appareil d'imagerie IRM 2 pourra par exemple comprendre un aimant de 1,5 Tesla, et sera apte à fournir des images de module (ou images anatomiques) et des images de phase (ou images thermiques), bidimensionnelles ou tridimensionnelles, de la région cible du tissu à traiter. On prendra de préférence un appareil d'imagerie IRM avec une résolution spatiale de l'ordre du millimètre, une précision de l'ordre de 1°C, et une résolution temporelle de l'ordre de la seconde.

Le dispositif de traitement 1 selon l'invention comprend en outre des moyens générateurs d'énergie sous la forme d'un transducteur matriciel 3 et d'un générateur multivoies 4 alimentant le transducteur matriciel 3. Le transducteur 3 est intégré dans le lit de l'aimant de l'appareil d'imagerie IRM 2 et il permet de focaliser une onde ultrasonore en direction d'un point de traitement P de la région cible. On prendra par exemple un transducteur matriciel de 256 éléments permettant de focaliser une onde ultrasonore de 1,5 MHz en un point de la taille de la longueur d'onde c'est-à-dire environ 1mm. La pression acoustique au point de traitement P et sa position sont ajustables par l'amplitude et le retard des signaux émis par le générateur multivoies 4. Ainsi, la position du point de traitement P peut être ajustée sur un volume de 15x15x30mm$^3$ toutes les 100 millisecondes.

Le transducteur matriciel 3 pourra être remplacé par un transducteur simple. Dans ce cas, le déplacement du point de traitement doit être fait de façon mécanique, avec un système de déplacement hydraulique par exemple, et présentera donc un temps de réponse plus lent que pour un transducteur matriciel.

Le dispositif de traitement thermique 1 comprend également une unité de contrôle 5 qui est apte à recevoir en entrée des données en provenance de l'appareil d'imagerie IRM 2 et, en fonction de ces données, à commander le générateur multivoies 4 pour modifier la position du point de traitement P via le transducteur matriciel 3.

En effet, les mesures acquises à l'intérieur de l'aimant de l'appareil d'imagerie IRM 2 sont transmises à des moyens de calcul 6 de l'unité de contrôle 5. Ces moyens de calcul 6 sont prévus pour traiter les données issues de l'appareil d'imagerie IRM 2 et les transmettre à des moyens de commande 7 de l'unité de contrôle 5. Les moyens de commande 7 utilisent les données issues des moyens de calcul 6 pour transmettre au générateur multivoies 4 les coordonnées et la puissance des prochains points cibles, par l'intermédiaire de fibres optiques par exemple. Le générateur multivoies 4 produit et amplifie les signaux électriques ultrasonores déphasés de sorte que le transducteur matriciel 3 émette une onde ultrasonore focalisée au point choisi. L'élévation de température induite à l'intérieur du point de traitement P permet ainsi d'obtenir la dose thermique souhaitée, nécessaire à l'obtention d'une nécrose par exemple.

Les moyens de calcul 6 sont donc prévus pour traiter les données issues de l'appareil d'imagerie IRM 2, en vue notamment d'estimer une position de la région cible.

La première étape de calcul consiste à convertir les mesures acquises à l'intérieur de l'aimant de l'appareil d'imagerie IRM 2 en images. A cette fin, on utilise un reconstructeur 8 d'images qui effectue par exemple une transformée de Fourier et des filtrages des différentes données issues des moyens d'imagerie IRM 2 de manière à reconstruire une image de la région cible.

Ces images permettant de visualiser la région cible étudiée sont ensuite transférées aux moyens de traitement 9 pour estimer la position de la région cible. En effet, le tissu biologique considéré étant en mouvement, la région cible à traiter l'est aussi, et il est donc nécessaire d'estimer de manière précise le déplacement de la région cible de manière à pouvoir corriger la position du point de traitement P de la région cible au cours du temps, en fonction du mouvement de la région cible. Une telle correction permettra de ce fait une irradiation plus précise des tissus, et par conséquent un traitement plus efficace.

Il existe diverses techniques d'imagerie pour estimer le mouvement des tissus biologiques à partir des images IRM, on pourra par exemple estimer le déplacement de ces tissus à partir d'images anatomiques construites à partir des données des moyens d'imagerie IRM 2. Plusieurs algorithmes de recalage d'images permettent en effet de faire correspondre les coordonnées de chaque point d'image à recaler avec les coordonnées de leurs homologues sur une image de référence.

En pratique, à cause des contraintes de temps réel, il est très difficile d'obtenir des images tridimensionnelles à cause des limitations techniques de la séquence d'acquisition nécessaire. Une approche alternative consiste donc à estimer le déplacement sur des images bidimensionnelles généré par des objets en mouvement dans un espace tridimensionnel. La position et l'orientation des coupes doivent être choisies en sorte que l'axe du mouvement appartienne au plan image. Si les estimations de déplacements de la région cible décrites ici se basent sur des images bidimensionnelles, l'invention n'est pas limitée aux estimations de champs de déplacements bidimensionnels, et pourra aisément être généralisée aux estimations de champs de déplacements tridimensionnels.

Parmi les différents algorithmes de recalage d'images, on peut distinguer plusieurs approches. Certains algorithmes se basent en effet sur l'estimation d'une transformation globale dans l'image alors que d'autres utilisent les informations relatives aux transformations locales de l'image. La méthode de recalage la plus efficace n'est pas celle qui donne la meilleure similarité entre l'image de référence et l'image recalée mais celle qui fournit une estimation du champ de déplacements la plus proche du mouvement réel des organes. La démarche utilisée consiste donc à s'affranchir dans un premier temps du mouvement global dominant, présent dans l'image, puis à affiner localement les déplacements des organes. Le but est donc d'estimer le mouvement à l'aide d'une approche locale en se basant sur les résultats obtenus par une approche globale.

La recherche des paramètres optimaux d'une transformation affine globale pourra par exemple être effectuée à l'aide d'une optimisation au sens des moindres carrés. Une approche hiérarchique de l'algorithme de Horn & Schunck permettra ensuite une bonne estimation du déplacement local des tissus car la contrainte de régularité, imposant que les vecteurs de déplacements doivent être similaires pour les pixels adjacents, coïncide avec le mouvement réel des tissus.

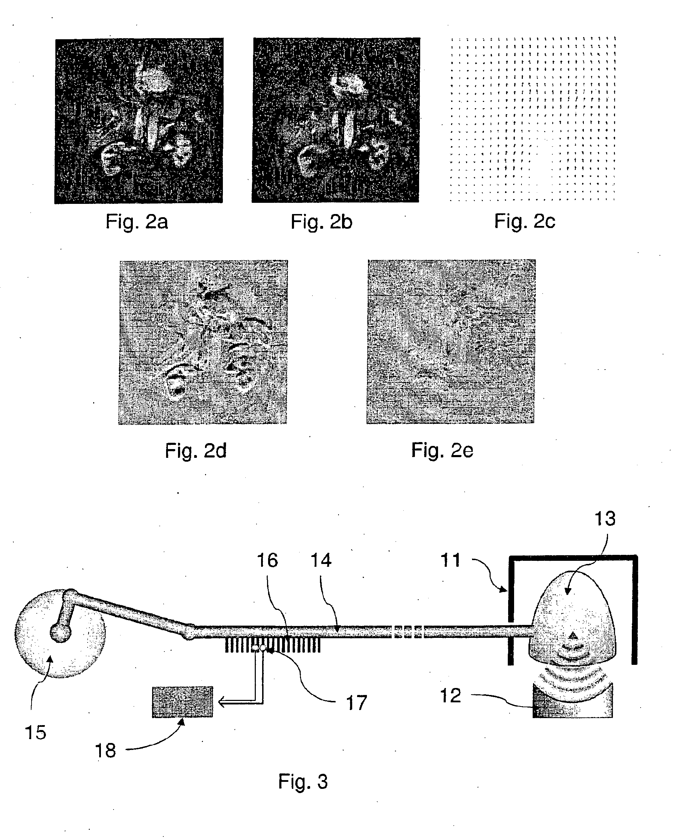

Les figures 2a à 2e illustrent ce processus de recalage d'images. Les figures 2a et 2b sont des images anatomiques de l'abdomen d'un humain en respiration libre, obtenues respectivement au début et à la fin de l'expiration. La figure 2c représente le champ de vecteurs bidimensionnel estimé à l'aide d'un algorithme de recalage d'images. La figure 2d est obtenue en soustrayant une image de référence (l'image de la figure 2a, au début de l'expiration) à l'image à recaler (l'image de la figure 2b, à la fin de l'expiration). La figure 2e représentant une image obtenue en soustrayant l'image de référence à l'image recalée, montre que le recalage d'image a été correctement effectué.

Ces différentes techniques de traitement d'images vont donc permettre, à partir des images résultant des données transmises par les moyens d'imagerie IRM 2, de déterminer de nombreuses informations concernant les mouvements de la région cible à traiter, ainsi qu'une estimation de la position de la région cible.

Lors d'une intervention sur un tissu biologique, ce dernier peut subir des déplacements et la position du point de traitement P doit être ajustée de telle sorte qu'il soit situé en permanence à l'intérieur de la région cible initialement définie. L'unité de contrôle 5 du dispositif de traitement 1 selon l'invention est donc prévue pour corriger les différents mouvements que le tissu biologique à traiter peut subir. Les mouvements du tissu biologique, et par conséquent de la région cible, peuvent se classer principalement suivant deux catégories, en fonction de leur occurrence temporelle ; il existe en effet les mouvements accidentels comme lors de la contraction d'un muscle par exemple et les mouvements dits périodiques, qui sont liés par exemple au cycle respiratoire ou au cycle cardiaque, ce sera le cas du foie et du rein. On notera que chacun de ces mouvements peut être vu comme un mouvement rigide (composé d'un mouvement de translation et/ou de rotation) bien qu'ils soient en fait des mouvements élastiques plus complexes.

Pour chacun de ces deux types de mouvements (accidentel ou périodique), il est nécessaire d'adapter la stratégie de correction à adopter.

En effet, pour corriger les mouvements accidentels par exemple, il convient d'utiliser par exemple le champ de vecteurs de déplacements estimé par les moyens de calcul à partir de la dernière image anatomique disponible. C'est grâce à cette estimation du champ de vecteurs de déplacements que la position du point de traitement P est corrigée en suivant le déplacement estimé relatif à sa localisation.

La redondance caractéristique d'un mouvement périodique permet d'adapter la stratégie de correction utilisée pour les mouvements accidentels de façon à ce que le positionnement du point de traitement P soit plus précis. En effet, la stratégie précédente impose que le mouvement réel de la région cible est assimilé au mouvement estimé sur la dernière image acquise. Selon cette hypothèse, on considère donc que l'intervalle de temps entre la transmission des données issues des moyens d'imagerie IRM 2 et la disponibilité des informations présentes dans cette image est négligeable. Ce délai de latence entre la transmission des données issues des moyens d'imagerie IRM 2 et la correction du positionnement du point de traitement P est typiquement composée du temps d'acquisition, du temps de reconstruction de l'image, du temps de transfert, et du temps de calcul pour estimer la position de la région cible. Ce délai étant typiquement d'environ 2 secondes, et la période d'un mouvement respiratoire d'environ 5 secondes, la stratégie adoptée pour corriger les mouvements accidentels génère un mouvement estimé en quasi opposition de phase avec le mouvement réel de la région cible. En conséquence, cette stratégie de correction peut doubler l'erreur de positionnement du point de traitement P si on l'utilise pour corriger les mouvements périodiques.

Pour ces raisons, il est indispensable de corriger le mouvement périodique en quantifiant le délai de latence à compenser: Une stratégie de correction peut par exemple se baser sur l'analyse de la variation du mouvement global dominant présent dans l'image.

La compensation d'un mouvement périodique nécessite plusieurs étapes qui sont décrites ci-après.

Il convient tout d'abord de modéliser analytiquement le mouvement dominant de la région cible à traiter. Cette étape de modélisation est réalisée par des moyens de modélisation opérant pendant une étape de prétraitement au cours de laquelle la séquence des mouvements du tissu biologique dé la région cible est analysée. Du fait du cycle respiratoire notamment, la région cible suit un mouvement présentant une certaine périodicité. Comme le cycle respiratoire n'est pas totalement régulier, une moyenne des périodes acquises pendant l'étape de prétraitement est effectuée de façon à établir une période type. Chacune de ces périodes est ajustée sur la période type établie de façon à obtenir un échantillonnage précis d'un cycle respiratoire.

On peut ensuite assimiler les mouvements dominants de la région cible en des mouvements périodiques se décomposant en série de Fourier d'ordre N sous la forme :

$$M(t) = \sum_{n=0}^{N} a_n \cdot \cos(n\omega t) + b_n \cdot \sin(n\omega t) \qquad \text{[Equation 1]}$$

Dans cette équation, $a_n$ et $b_n$ sont les composantes de chaque harmonique. En pratique $N$=3 se trouve être un bon compromis pour modéliser efficacement une période type. Les harmoniques d'ordre supérieur ont en effet une amplitude inférieure à 0,1 millimètre.

La détermination des coefficients $a_n$ et $b_n$ se fait à partir de la méthode des moindres carrés avec les $K$ différents points acquis durant l'étape de prétraitement :

$$a_n = \frac{\sum_{i=1}^{K} M(t_i)\cos(n\omega t_i)}{\sum_{i=1}^{K} \cos(n\omega t_i)^2} \quad \text{et} \quad b_n = \frac{\sum_{i=1}^{K} M(t_i)\sin(n\omega t_i)}{\sum_{i=1}^{K} \sin(n\omega t_i)^2} \qquad \text{[Equation 2]}$$

Selon un mode de réalisation particulier de l'invention, les moyens de modélisation sont en outre aptes à modéliser le mouvement de la région cible pendant l'étape de traitement.

En outre, on peut envisager des moyens de modélisation aptes à modéliser des mouvements non périodiques, ces mouvements étant caractérisés en ce qu'ils s'étendent sur une durée relativement longue, en comparaison des mouvements accidentels.

Le mouvement de la région cible étant modélisé, on pourra corriger de manière beaucoup plus précise la position du point de traitement P.

La première étape en vue du repositionnement du point de traitement P consiste à analyser le signal de mesure issu des moyens d'imagerie IRM 2, de la manière décrite précédemment par exemple, de façon à obtenir des informations sur la position de la région cible.

Il convient en effet tout d'abord de déterminer la position spatiale de la région cible à partir de l'image anatomique issue des moyens d'imagerie IRM 2. La position spatiale découle de l'analyse du déplacement $D$ de la région cible entre une image et l'image de référence.

La deuxième étape consiste à déterminer la position temporelle correspondant à l'image de la région cible. Cette position temporellé $t_i$ de la dernière image est déterminée en fonction de la position spatiale précédemment déterminée, et par voie de conséquence du déplacement dominant $D$ correspondant sur la période type , en déterminant la solution de l'équation $D = M(t_i)$.

Néanmoins, le polynôme trigonométrique $M(t)$ modélisant le mouvement de la région cible possède plusieurs racines ; une indétermination existe donc en ce qui concerne la période ascendante ou descendante du cycle. Pour lever cette indétermination, il est donc nécessaire de se référer à l'historique des déplacements de la région cible. On pourra par exemple calculer une distance euclidienne entre le déplacement dominant des $L$ dernières dynamiques (avec par exemple $L$ fixé à 5) et le polynôme trigonométrique pour localiser temporellement de manière stable et précise la position de la région cible sur l'image acquise.

Une fois la position temporelle déterminée, la position de la région cible par rapport à la mobilisation du mouvement est précisément définie. Néanmoins, cette position estimée correspond à la position de la région cible au moment où les moyens d'imagerie IRM 2 ont fait l'acquisition d'une image, et pas à la position réelle de la région cible. En effet, pendant le délai de latence qui a été nécessaire pour transférer et traiter les données issues des moyens d'imagerie IRM 2, le mouvement de la région cible n'a pas cessé. La position réelle de la région cible au moment où les informations de position sont disponibles est différente de la position ainsi estimée. Il convient donc de compenser le mouvement qui a eu lieu pendant ce délai de latence.

Ainsi, les moyens de commande 7 de l'unité de contrôle 5, destinés à positionner le point de traitement P, sont aptes à déterminer la position réelle de la région cible et de positionner le point de traitement P en fonction de cette position réelle.

Dans un premier temps, les moyens de commande déterminent la position temporelle réelle qui correspond à la position temporelle estimée à laquelle vient s'ajouter le délai de latence nécessaire aux calculs de position.

Une fois la position temporelle réelle déterminée, on utilise la modélisation du mouvement de la région cible pour déterminer la position spatiale réelle correspondante, et positionner le point de traitement P en fonction de cette position spatiale réelle.

Pour déterminer la position temporelle réelle, on pourra par exemple considérer que le délai de latence est constant, en le prenant égal à la valeur moyenne des délais de latence mesurés pendant l'étape de prétraitement.

Selon un autre mode de réalisation de l'invention, le dispositif de traitement 1 sera pourvu de moyens de mesure 10 pour mesurer précisément la valeur du délai de latence de chaque dynamique nécessaire à l'estimation de position de la région cible à partir des données des moyens d'imagerie IRM 2. Le dispositif de traitement 1 pourra par exemple comprendre un microcontrôleur 10 pour chronométrer le temps entre le moment où les données des moyens d'imagerie IRM 2 sont reçues par les moyens de calcul 6 et le moment où la position temporelle estimée est déterminée. Comme on le verra plus loin, l'utilisation de tels moyens de mesure 10 permettent une compensation précise du délai de latence, ce dernier dépendant très fortement des différents temps de calcul opérés par les moyens de calcul, ces temps de calcul pouvant varier d'une estimation à une autre, du fait que leur capacité de travail peuvent varier d'une dynamique à une autre.

Outre la compensation du mouvement de la région cible pendant le délai de latence, les moyens de commande

7 sont aptes à anticiper le mouvement de la région cible.

Il existe en effet un certain délai entre deux dynamiques successives d'acquisition de données par les moyens d'imagerie IRM 2, et donc un délai entre deux déterminations successives de la position spatiale réelle de la région cible. Néanmoins, la connaissance de la position temporelle estimée de la région cible et du délai de latence, mesuré par exemple par un microcontrôleur 10, permet d'anticiper les déplacements dominants à venir jusqu'au traitement des données IRM suivantes, grâce au polynôme modélisant le mouvement dominant de la région cible.

Si l'on cherche à repositionner le point de traitement P dans la région cible alors que s'est écoulé un certain temps depuis la dernière détermination de la position spatiale réelle de la région cible, temps qu'on appelle « délai d'anticipation », il suffit de déterminer la position spatiale anticipée correspondant sur la modélisation du mouvement de la région cible à une position temporelle anticipée, cette position temporelle anticipée étant égale à la dernière position temporelle estimée augmentée du délai de latence et du délai d'anticipation.

On choisira de préférence plusieurs délais anticipés de façon à repositionner le point de traitement le plus souvent possible. De préférence encore, les moyens de commande repositionneront le point de traitement P de manière anticipée jusqu'à ce qu'une nouvelle position temporelle estimée soit déterminée par les moyens de calcul 6.

Il est à noter que dans tous les cas où le délai de latence est très faible, du fait des performances des moyens de calcul 6 par exemple, il pourra être envisagé de le négliger. Dans ce cas, les moyens de commande 7 pourront compenser le mouvement de la région cible en positionnant le point de traitement P en tenant compte du délai d'anticipation uniquement (le délai de latence étant considéré comme nul).

Pour les mouvements de tissus considérés comme rigides, la connaissance du mouvement global dominant suffit à déterminer le champ de déplacements sur l'ensemble de l'objet et la modélisation du mouvement dominant est donc aussi suffisante.

En revanche, pour les mouvements plus complexes de tissus, tels que des mouvements élastiques, un atlas de mouvement est construit. Cet atlas contient l'historique des champs de déplacements (tel que celui présenté figure 2c) estimés pendant une étape de prétraitement effectuée avant l'intervention. Pendant l'intervention, le mouvement global dominant le plus proche est recherché dans l'atlas pour en déduire le champ de déplacements locaux associés. C'est sur la base des champs de déplacements que les estimations de position vont être effectuées.

En outre, pour augmenter la stabilité du système, il convient de réduire au maximum le délai de latence. Pour ce faire, on pourra utiliser d'autres moyens de mesures délivrant des signaux temps réel de mesure de la région cible (comme par exemple les échos navigateurs ou les échos ultrasonores) de façon à définir le mouvement dominant de la cible. Ce mouvement dominant peut être utilisé pour corriger directement les mouvements rigides ou permettre la sélection des champs de déplacements dans l'atlas créé à partir des images IRM.

Les signaux temps réel de mesure de la région cible permettant d'obtenir une estimation du mouvement avec une excellente résolution temporelle et les signaux de mesure IRM fournissant quant à eux une estimation spatiale précise du mouvement, une approche combinant les différents signaux de mesure de la région cible permettrait donc de tirer parti des avantages spatiaux et temporels de chaque outil de mesure du mouvement.

Selon un autre mode particulier de réalisation de l'invention, les moyens d'imagerie IRM 2 du dispositif de traitement sont non seulement capables de fournir des données relatives à une image anatomique de la région cible, mais ils sont également prévus pour fournir des données relatives à une image thermique de la région cible. Une telle image thermique permet de représenter une distribution spatiale de la température de la région cible.

L'imagerie par résonance magnétique est en effet basée sur la détection des propriétés magnétiques des protons contenus dans les molécules d'eau du corps. Le système de formation des images par résonance magnétique associe chaque unité de volume (voxel) de la région étudiée à un nombre complexe $Me^{i\varphi}$, où M est le module et $\varphi$ la phase du vecteur d'aimantation macroscopique. Le module $M$ donne l'information anatomique et permet la construction de l'image dite anatomique. Le principe de la thermométrie guidée par IRM est d'effectuer une acquisition dynamique des images en analysant les variations de contrastes pour calculer les cartographies de température. La méthode la plus couramment utilisée, notamment à haut champ (1,5 Tesla), pour effectuer une mesure dynamique de température est d'effectuer la comparaison de contraste de phase d'images obtenues à des temps différents. Dans certaines conditions bien définies (sans artefact de mouvement et sans susceptibilité), une différence de phase entre deux images consécutives est directement proportionnelle à une différence de température :

$$\Delta\varphi = \gamma\,\alpha.B_0.\Delta T.T_E \qquad\qquad \text{[Equation 3]}$$

où $\Delta T$ est la différence de température, $\gamma$ le rapport gyromagnétique ($\gamma \approx 42,58.2\pi$ MHz/T), $\alpha$ le coefficient de température (=0.01 ppm/K), $T_E$ le temps d'écho, $B_0$ l'induction magnétique de l'aimant. Ce calcul est effectué en chaque pixel de l'image pour obtenir des cartes de température.

Avec un tel suivi de la thermométrie de région cible, il sera possible de déterminer la dose thermique déposée au cours du traitement, de manière à pouvoir évaluer le niveau de nécrose de la région cible du tissu biologique à traiter.

Néanmoins, cette méthode de suivi de la thermométrie est sensible aux mouvements que peut subir la région cible du tissu biologique. Il est donc nécessaire, pour un suivi précis des variations de températures, de corriger les artefacts de thermométrie générés par les différents mouvements de la région cible, qu'ils soient accidentels ou périodiques.

En effet, si un mouvement survient accidentellement entre les instants $t_{n-1}$ et $t_n$, les cartographies de température calculées après ce mouvement accidentel seront erronées. Une méthode simple consiste à prendre l'image de phase acquise à l'instant $t_n$ ($\varphi_n$) comme nouvelle image de phase de référence. La carte de température à l'instant $t_i$ avec $i > n$, est calculée avec l'équation :

$$\Delta T_i = \Delta T' + (\varphi_i - \varphi_n).k \qquad\qquad \text{[Equation 4]}$$

où $\Delta T'$ est la n-1 cartographie de température après correction du mouvement.

Pour corriger les artefacts de thermométrie générés par des mouvements périodiques, il est possible d'analyser la perturbation des images thermiques en fonction du mouvement lors d'une étape de prétraitement effectuée avant l'intervention. Un atlas de mouvements est construit à partir des images acquises pendant l'étape de prétraitement, qui comporte la même séquence que l'intervention mais au cours de laquelle aucune hyperthermie n'est pratiquée. On fera l'acquisition de 50 images par exemple pour permettre un échantillonnage précis du cycle respiratoire. L'image de phase de référence choisie est la première de la série temporelle. Pendant l'étape de prétraitement, les images anatomiques sont stockées dans un atlas avec l'image de phase correspondante. Pendant l'intervention, l'image anatomique courante est comparée aux images anatomiques stockées dans l'atlas. L'image dans l'atlas la plus similaire est sélectionnée et l'image de phase correspondante est choisie comme référence pour le calcul de la température. Le déplacement des organes est ensuite estimé sur les images anatomiques afin d'être compensé à la fois sur les images anatomiques et les images de température.

En outre, quand le suivi des variations de température et donc de la dose thermique appliquée à la région cible, est réalisé de manière précise, c'est-à-dire en corrigeant les artefacts de thermométrie dues aux différents mouvements de la région cible, on pourra prévoir au sein du dispositif de traitement 1 des moyens de régulation du rayonnement appliqué au point de traitement P dans la région cible.

Les images thermiques issues de moyens d'imagerie IRM 2 permettant de suivre la dose thermique ayant été appliquée jusqu'alors à la région cible, il pourra être intéressant de réguler le rayonnement irradiant la région cible au point de traitement P, de manière à ce que la distribution spatiale de température de la région cible soit conforme à une consigne de distribution spatiale de température.

Les informations thermométriques issues des images thermiques notamment permettent donc de contrôler précisément la température de la région cible, et de la réguler par l'intermédiaire des moyens de régulation de manière à ce qu'elle soit conforme à la température désirée en vue de la nécrose de la région cible du tissu biologique par exemple.

Lorsque la région cible peut être traitée par traitement ponctuel, la température peut être ajustée à partir des images thermiques IRM par une régulation automatique Proportionnel, Intégral et Dérivé (PID). Cette technique est basée sur l'équation différentielle [Equation 5] incluant un terme proportionnel, dérivé et intégral pour minimiser l'erreur de température $\xi$.

$$\frac{\partial \xi}{\partial t} + a\xi + \frac{a^2}{4}\int_0^t \xi = 0 \quad \text{avec} \quad \xi = \theta - T \qquad \text{[Equation 5]}$$

Le paramètre $\xi$ représente la différence entre la température cible $\theta$ et la température mesurée T.

Le terme proportionnel est égal à l'erreur instantanée entre la température mesurée et la température cible. Le terme intégral est défini par la somme de l'erreur sur la température passée. Le terme dérivé est lui déterminé par la variation présente sur la température de façon à atteindre la température voulue.

Le paramètre a peut être défini par l'opérateur et influence l'importance relative des trois termes dans le contrôle PID. Ce contrôle automatique assure une convergence stable vers la température cible.

Le contrôle PID est aussi combiné avec l'équation de transfert thermique [Equation 6] de façon à anticiper la réaction du tissu biologique. Cette équation prend en compte la diffusion thermique et l'absorption de l'onde ultrasonore dans le tissu.

$$\frac{\partial T}{\partial t} = D \cdot \nabla^2 T + \alpha \cdot P \qquad \text{[Equation 6]}$$

L'équation différentielle PID est respectée si la puissance est choisie selon la valeur définie par l'expression [Equation 7] de façon à ajuster correctement le terme dérivé de la température :

$$P = \frac{1}{\alpha}\left[\frac{\partial T_C}{\partial t} - D \cdot \nabla^2 T + a\left(\theta - T\right) + \frac{a^2}{4}\int_t\left(\theta - T\right)\right] \qquad \text{[Equation 7]}$$

Pour contrôler la température sur un point déterminé du tissu mobile, l'algorithme d'asservissement PID est calculé avec les cartes de températures corrigées qui sont recalées sur une position centrale de référence. Avec cette carte de température, tous les calculs concernant la puissance requise pour le contrôle de la température sont fait de la même façon que pour un tissu biologique statique. A chaque positionnement du point de traitement, qu'il soit mesuré ou anticipé, la puissance du rayonnement pourra donc être ajustée.

Pour traiter un large volume de plusieurs millilitres avec un petit point de traitement de seulement un millimètre, une solution consiste à déplacer le point de traitement selon une trajectoire. Pour un contrôle spatial de la température, la quantité de puissance acoustique est définie par l'équation [Equation 7] pour tous les points du volume cible. Cependant, quel que soit le type de technologie utilisée pour déplacer le point de traitement P (électronique ou mécanique), il est techniquement difficile d'émettre cette puissance définie simultanément en chacun des points. Pour éviter ce problème, la quantité d'énergie déposée $E$ durant un cycle d'asservissement de durée $t_F$ en un point $r$ est définie par l'équation [Equation 8].

$$E_{(\bar{r})} = \frac{t_F}{\alpha}\left[\frac{\partial \theta_{(\bar{r})}}{\partial t} - D \cdot \nabla^2 T_{(\bar{r})} + a\left(\theta_{(\bar{r})} - T_{(\bar{r})}\right) + \frac{a^2}{4}\int_t\left(\theta_{(\bar{r})} - T_{(\bar{r})}\right)\right]$$

$$\text{[Equation 8]}$$

Cette énergie est délivrée successivement en chaque point, en choisissant la durée et la puissance appropriée. Les points nécessitant un haut niveau énergétique sont chauffés pendant une longue durée de focalisation plutôt qu'avec une puissance élevée de manière à améliorer la sécurité. Ainsi la durée d'un cycle d'asservissement est divisée en plusieurs durées avec une pondération correspondant à l'énergie requise de façon à limiter la puissance maximale émise. Pour optimiser la qualité du contrôle de la température, le cycle d'asservissement est choisi le plus court possible, soit la durée d'acquisition d'une dynamique.

Les moyens de commandes forçant le point de traitement P à suivre le mouvement de la région cible, de manière calculée ou anticipée, la trajectoire que le point de traitement P doit suivre pour que le traitement d'un large volume soit possible est aussi modifiée. Chaque point définissant cette trajectoire est corrigé de la même manière qu'il a été décrit précédemment, par les moyens de commandes. De cette façon, le rayonnement régulé est échantillonné correctement, et permet un traitement volumétrique efficace.

### *Evaluations de traitements de tissus biologiques en mouvement avec un dispositif selon l'invention*

Comme illustré sur la figure 3, le dispositif de traitement selon l'invention a été évalué sur un muscle de porc ex-vivo, rendu mobile artificiellement, placé dans l'appareil d'imagerie IRM 11 et traité par ultrasons focalisés à l'aide d'un transducteur 12. La région cible 13 est déplacée à distance par un arbre de transmission 14. Les mouvements simulés sont alors accidentels si l'arbre 14 est déplacé manuellement ou périodiques s'il est entraîné par un moteur 15. En faisant varier la tension d'alimentation du moteur 15, il est alors possible de choisir la période du mouvement. De plus le mouvement de la région cible 13 peut être rigide si elle est placée sur un support glissant ou élastique si elle est en face d'une butée.

Dans le cas de l'étude du mouvement rigide, translation de la région cible 13, la position de l'arbre de transmission 14 est mesurée par le biais d'une barrette graduée 16 tous les millimètres. Deux photos diodes 17 émettrices et deux photos récepteurs permettent de relever la position de cette règle avec une précision de 0,5mm. Le décomptage des graduations est effectué par un microcontrôleur PIC 18 à 20MHz qui chronomètre à la microseconde près le déplacement de la cible. A chaque changement de position de la règle graduée, l'heure et la position de la cible sont transmises à la console de monitorage en quelques microsecondes, par le biais d'une connexion RS232 par exemple. Ces relevés en temps réel de la position de la cible 13 servent de référence pour évaluer la qualité du déplacement estimé et anticipé à partir des images provenant de l'IRM.

*Correction de la position du point de traitement*

Il convient dans un premier temps d'évaluer les performances du dispositif de traitement en ce qui concerne la correction des mouvements de la région cible. Il est intéressant de comparer le mouvement estimé (c'est-à-dire sans compensation du délai de latence) sur les images IRM et celui anticipé (c'est-à-dire à la fois le mouvement anticipé et le mouvement compensé prenant en compte le délai de latence) avec le mouvement réel mesuré par un microcontrôleur sur la réglette graduée disposé sur l'arbre de transmission.

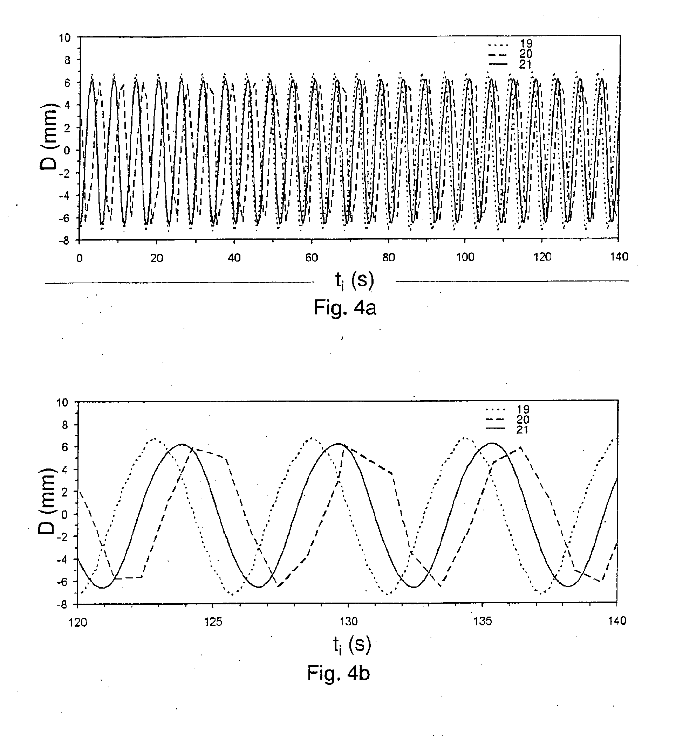

La première technique consiste à supposer que la période du mouvement est parfaitement constante au cours de l'intervention. Une approximation polynomiale d'ordre 3 du déplacement dominant anticipé sur l'étape de prétraitement est utilisée pour corriger le mouvement. Ce polynôme coïncide bien avec le mouvement réel sur les premières dynamiques. Néanmoins, malgré la variation de la vitesse angulaire du moteur inférieure à 1%, le mouvement anticipé modélisé par une fonction polynomiale non réactualisée diverge rapidement du mouvement réel. Les figures 4a et 4b présentent le déplacement réel (courbe 19) mesuré sur la règle graduée, le déplacement estimé (courbe 20) sur les images anatomiques ainsi que le déplacement anticipé (courbe 21) modélisé par une fonction polynomiale constante.

L'écart-type entre le mouvement réel et le mouvement anticipé est très faible au début de l'expérience (0,33mm) augmente rapidement jusqu'à 4,5mm au bout de 2 minutes. Cette technique démontre qu'il est impératif de réactualiser l'anticipation du mouvement le plus fréquemment possible.

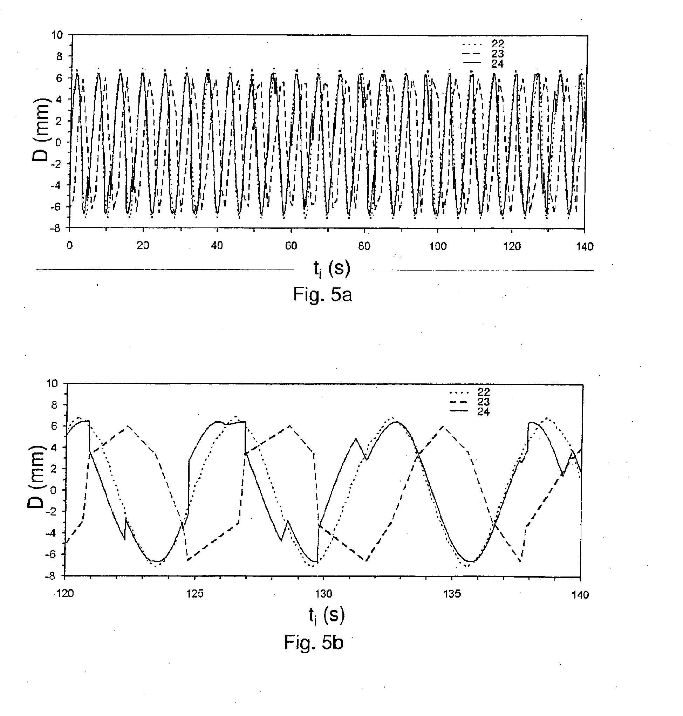

Selon une autre évaluation de la correction, on considère que le délai de latence est constant. Le muscle ex-vivo a été soumis dans cette expérience à un mouvement de translation périodique d'amplitude 14mm et de période environ égale à 5,6s. Sur la plateforme de test utilisée le délai de latence moyen à compenser est d'environ 1,9s. Sur les figures 5a et 5b illustrant cette évaluation, la courbe 22 représente le déplacement réel mesuré sur la règle graduée, la courbe 23 le déplacement estimé sur les images anatomiques, et la courbe 24 le déplacement anticipé réactualisé à chaque dynamique avec une valeur de délai de latence constante.

Cette méthode est plus efficace que la précédente car l'écart-type mesuré entre le mouvement mesuré et anticipé oscille entre 1mm au début de l'expérience et 2mm à la fin.

Le délai à compenser est principalement composé de la durée d'acquisition d'une dynamique (1s) et du délai de transmission et de calcul variant de 0,3s à 1,1s (0,9 s en moyenne). Cette variation du temps de traitement des informations est en partie liée à l'utilisation d'un système d'exploitation qui n'est pas temps réel. Cette variation du délai de transmission induit une perturbation de la localisation temporelle du mouvement estimé qui se répercute sur le mouvement anticipé. Ainsi le mouvement anticipé est composé d'une succession d'arches trigonométriques disjointes.

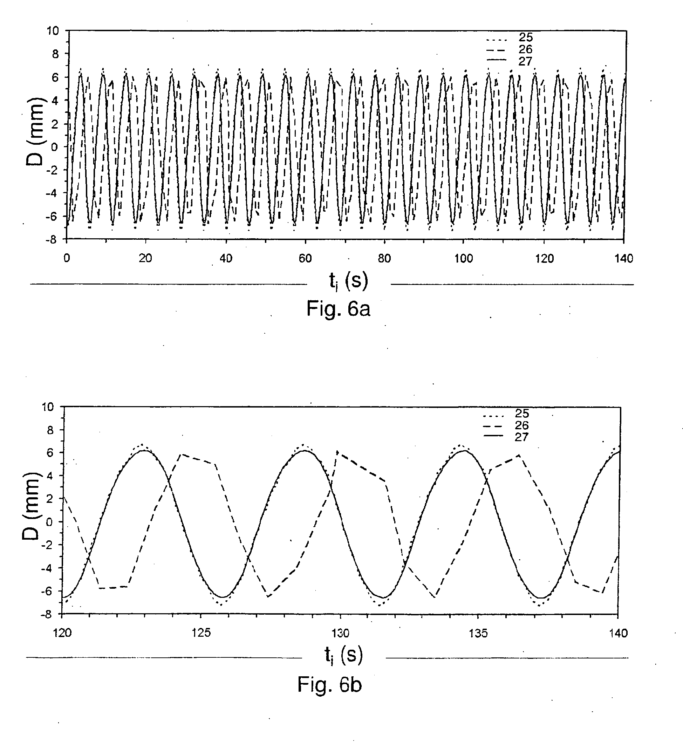

Selon encore une autre évaluation de la correction, le délai de latence à compenser est obtenu en comparant l'heure du début de la dernière dynamique chronométrée par un microcontrôleur avec l'heure de la fin de son traitement. De la même façon que précédemment, le muscle ex-vivo a été soumis à un mouvement de translation périodique d'amplitude 14mm et de période environ égale à 5,6s. Sur les figures 6a et 6b illustrant cette évaluation, la courbe 25 représente le déplacement réel mesuré sur la règle graduée, la courbe 26 le déplacement estimé sur les images anatomiques, et la courbe 27 le déplacement anticipé réactualisé à chaque dynamique avec la valeur du délai de latence mesurée.

Cette méthode est très efficace car l'écart-type mesuré entre le mouvement mesuré et anticipé est de 0,33mm. Le mouvement anticipé est continu à l'exception d'infimes discontinuités permettant l'ajustement de la période du mouvement.

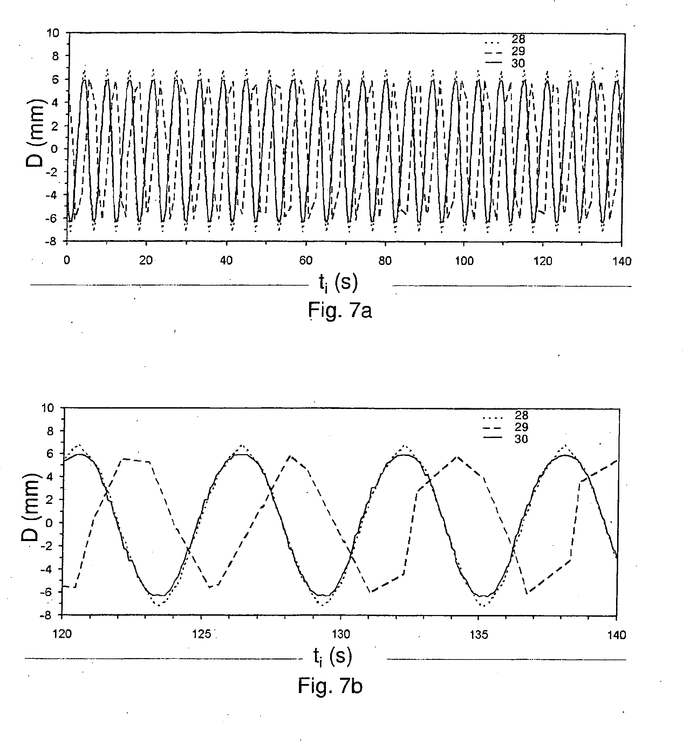

Comme indiqué plus haut, pour corriger un mouvement élastique, le déplacement dominant ne suffit plus. Il va néanmoins servir de critère de sélection du champ de vecteurs approprié dans l'atlas. Les figures 7a et 7b montrent le mouvement anticipé obtenu en utilisant la technique de référence à un atlas sur le mouvement de translation précédemment étudié, avec un délai de latence qui est mesuré à chaque dynamique. La courbe 28 représente le déplacement réel mesuré sur la règle graduée, la courbe 29 le déplacement estimé sur les images anatomiques, et la courbe 30 le déplacement anticipé.

Le déplacement anticipé est discrétisé sur 50 valeurs correspondant aux 50 champs de vecteurs stockés dans l'atlas. Cette discrétisation du mouvement anticipé diminue très légèrement la précision de la détermination du mouvement. L'écart-type entre le mouvement réel et le mouvement anticipé est de 0,41mm au lieu de 0,33mm obtenu en utilisant le mouvement dominant.

On constate avec ces différentes évaluations que quand aucune correction n'est effectuée, l'erreur moyenne commise de positionnement du point de traitement mesurée expérimentalement est de 4,76mm.

L'estimation théorique de cette erreur peut se faire en approximant le mouvement réel par sa première harmonique et en décalant l'origine temporelle. L'équation [Equation 1] s'écrit alors :

$$M(t) \approx c_1 \cdot \cos(wt) \quad \text{avec} \quad c_1 = \sqrt{a_1 + b_1} = 6,5\,mm \quad \text{et} \quad T = \frac{2\pi}{\omega} = 5,6\,s$$

[Equation 9]

L'erreur théorique induite par ce mouvement est donc :

$$\sigma_{\text{sans correction}} = \sqrt{\frac{1}{T} \int_0^T (c_1 \cdot \cos(wt))^2 \, dt} = \frac{c_1}{\sqrt{2}} = 4,6mm$$

[Equation 10]

Lorsque le point de focalisation est positionné selon le mouvement estimé sur la dernière image disponible, l'erreur expérimentale est en moyenne de 7,54mm.

L'estimation théorique de cette erreur peut être effectuée en supposant le délai de latence à compenser constant (1,9s). Le déphasage induit est :

$$\varphi_C = 2\pi \frac{d_C}{T'} = 2,1\,rad = 120°$$

[Equation 11]

L'erreur théorique induite par ce déphasage est :

$$\sigma_{\text{sans anticipation}} = \sqrt{\frac{1}{T} \int_0^T (c_1 \cdot \cos(wt) - c_1 \cdot \cos(wt + \varphi_C))^2 \, dt} = c_1 \cdot \sqrt{2} \cdot \left| \sin\frac{\varphi}{2} \right| = 8mm$$

[Equation 12]

Lorsque le délai de latence à compenser est correctement mesuré, l'erreur expérimentale mesurée est de 0,33mm. En théorie il n'y a plus de déphasage entre le mouvement anticipé et le mouvement réel :

$$\sigma_{\text{anticipé}} = 0$$

[Equation 13]

Le tableau suivant compare les écarts types théoriques et expérimentaux obtenus :

| Ecart type | Sans correction | Correction sans anticipation | Correction avec anticipation |
|---|---|---|---|
| Expérimental | 4,76 mm | 7,54 mm | 0,33 mm |
| Théorique | 4,6 mm | 8 mm | 0 mm |

Le mouvement anticipé permet une précision de positionnement du point de traitement au moins 14 fois plus précise qu'un positionnement sans correction du mouvement.

Notons enfin que la correction des mouvements périodiques se limite à la correction des mouvements ayant une période plus grande que la durée d'une acquisition d'une dynamique. En effet, d'après le théorème de Shanon-Nyquist un échantillonnage d'au moins deux dynamiques par période est nécessaire afin de pouvoir reconstruire une période complète. Dans les expériences présentées, la période du mouvement effectué était d'environ 5,6 secondes, l'acquisition d'une dynamique par seconde a donc permis une bonne reconstitution du mouvement.

*Correction des artefacts de thermométrie en plus de la correction de la position du point de traitement*

Il convient ensuite d'évaluer les performances du dispositif de traitement lorsqu'il est en outre apte à corriger les artefacts de thermométrie dus aux mouvements de la région cible. On a donc chauffé le muscle ex-vivo à l'aide du dispositif d'ultrasons focalisés tout en le soumettant successivement à différents types de mouvement au cours du chauffage.

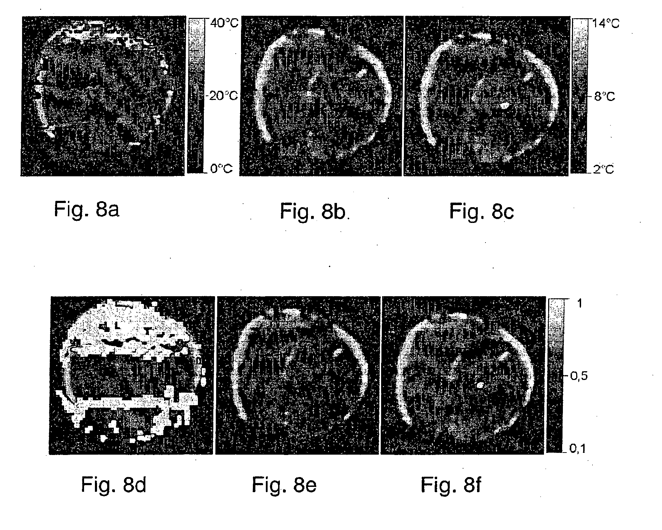

Le muscle ex-vivo a dans un premier temps été soumis à un mouvement de translation accidentel de 14mm. Le muscle a été chauffé par le transducteur à ultrasons émettant une puissance de 75W électrique pendant 50 secondes. Le mouvement accidentel a été effectué à mi-temps de la durée du chauffage. Les figures 8a à 8f permettent de comparer des cartographies de température (pendant le chauffage) et de dose thermique (à la fin de l'expérience). Les figures 8a à 8c représentent en effet les cartographies de température respectivement sans correction de la thermométrie et de la position du point de traitement, avec correction de la thermométrie mais sans correction de la position du point de traitement, et enfin avec correction de la thermométrie et de la position du point de traitement. De la même façon, les figures 8d à 8f représentent les cartographies de dose thermique respectivement sans correction de la thermométrie et de la position du point de traitement, avec correction de la thermométrie mais sans correction de la position du point de traitement, et enfin avec correction de la thermométrie et de la position du point de traitement.

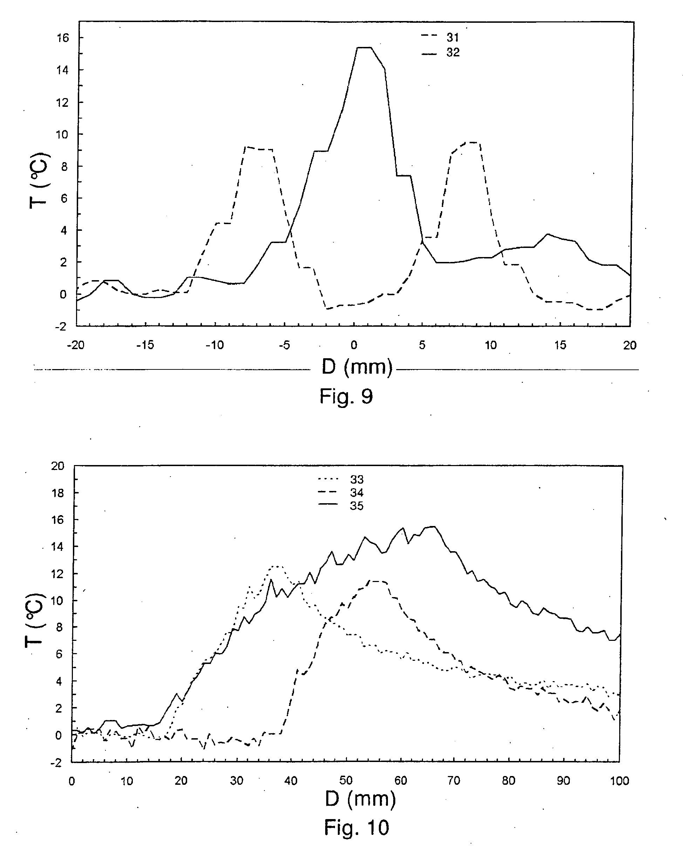

La figure 9 montre quant à elle la répartition spatiale de la température le long de l'axe vertical passant par les points focaux présentés sur les cartographies de température, avec (courbe 32) et sans (courbe 31) correction de la position du point de traitement.

La Figure 10 montre l'évolution temporelle de la température dans les zones chauffées au cours de cette expérience avec (courbe 35) et sans (courbe 33 avant mouvement, courbe 34 après mouvement) correction de la position du point de traitement.

L'erreur de température, commise sans correction de la thermométrie, peut atteindre 40°C. Cet artefact masque complètement le chauffage effectué. De même la dose thermique calculée est inexploitable.

Si aucune correction du point de traitement n'est effectuée, deux zones chauffées apparaissent simultanément : l'ancienne zone chauffée en cours de refroidissement et la nouvelle zone ciblée en cours de chauffage. Ces deux zones sont espacées de 14mm conformément au mouvement effectué. Aucune d'elles ne mène à une nécrose tissulaire car l'accumulation locale d'énergie n'a pas été suffisante.

Lorsque le déplacement du point de traitement compense le mouvement de la cible, une seule zone chauffée est observée. La forme logarithmique continue de la montée en température montre que le chauffage n'est pas affecté par le mouvement. De même, sa forme circulaire indique que le mouvement a été correctement corrigé. L'énergie étant toujours déposée sur la même région tissulaire, une nécrose a été induite.

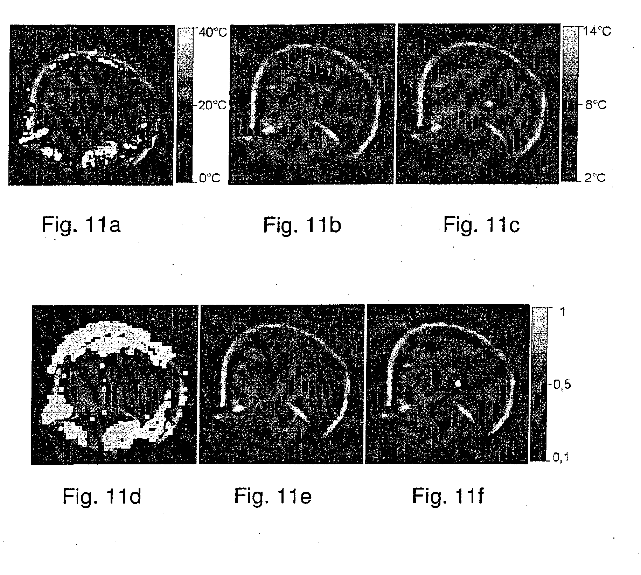

Le muscle ex-vivo a ensuite été soumis à un mouvement de translation périodique d'amplitude 14mm de période environ égale à 5,6s. Le muscle a été chauffé par le transducteur à ultrasons émettant une puissance de 100W électrique pendant 1 minute. Les figures 11a à 11f permettent de comparer les cartographies de température (figures 11a à 11c) et de dose thermique (figures 11d à 11f), sans correction de la thermométrie et de la position du point de traitement (figures 11a et 11d), avec correction de la thermométrie mais sans correction de la position du point de traitement (figures 11b et 11e), et enfin avec correction de la thermométrie et de la position du point de traitement (figures 11c et 11f).

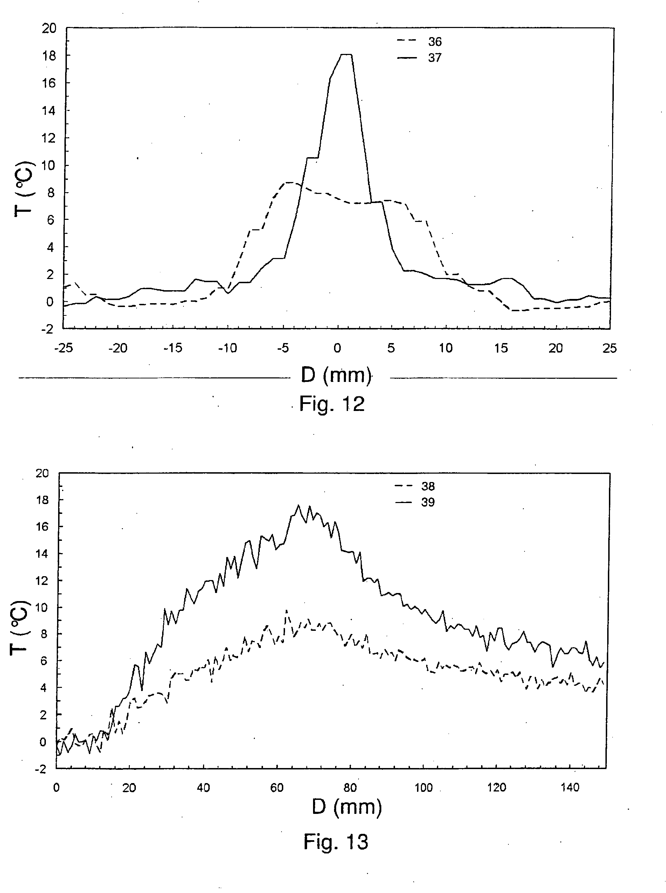

La Figure 12 montre la répartition spatiale de la température le long de l'axe vertical. (correspondant à l'axe du mouvement effectué par le muscle) passant par les zones de chauffage présentées sur les cartographies de température, avec (courbe 37) et sans (courbe 36) correction de la position du point de traitement.

La Figure 13 montre l'évolution temporelle de la température dans les zones chauffées au cours de cette expérience avec (courbe 39) et sans (courbe 38) correction de la position du point de traitement.

Comme précédemment, l'erreur de température commise sans correction de la thermométrie peut atteindre 40°C. Cet artefact masque complètement le chauffage effectué. De même la dose thermique calculée est inexploitable.

Lorsque aucune correction du point de focalisation n'est effectuée, la zone de chauffage est étalée sur 14mm. Le chauffage induit ne mène pas à une nécrose tissulaire car la montée en température est insuffisante.

Lorsque le déplacement du point de focalisation compense le mouvement de la cible, une zone de chauffage circulaire indique que le mouvement a été correctement corrigé. La forme logarithmique continue de la montée en température montre que le chauffage n'est pas affecté par le mouvement. La température atteinte par le tissu est deux fois plus élevée que précédemment. L'énergie étant toujours déposée sur la même région tissulaire, une nécrose a été induite. ,

Le cas le plus fréquent et le plus complexe à corriger est le cas des mouvements élastiques périodiques. Pour étudier ces déplacements, le muscle ex-vivo a été soumis à un mouvement d'écrasement périodique de période environ égale à 5.6s. Le muscle a été chauffé par le transducteur à ultrasons émettant une puissance de 100W électrique pendant 1 minute.

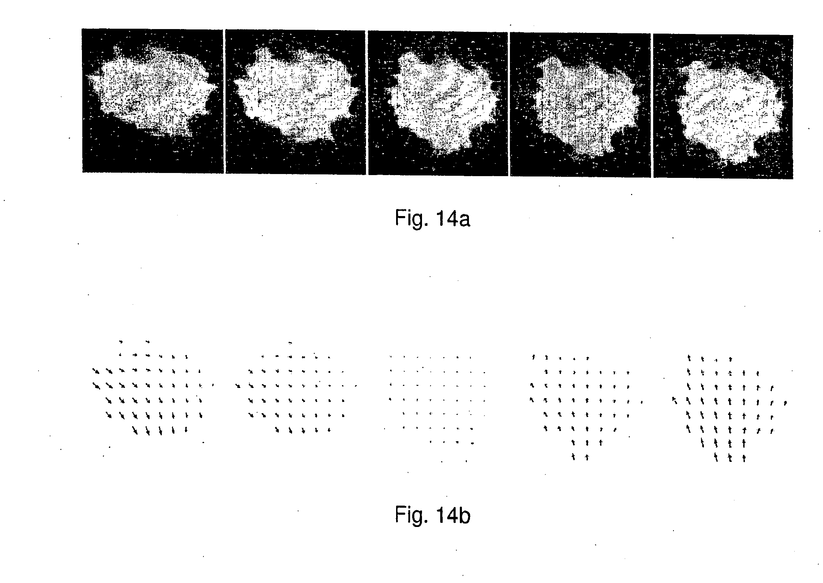

Les figures 14a et 14b montrent respectivement les images anatomiques obtenues à différentes positions du mouvement et les champs des vecteurs de déplacements associés, estimés par rapport à une image centrale de référence.

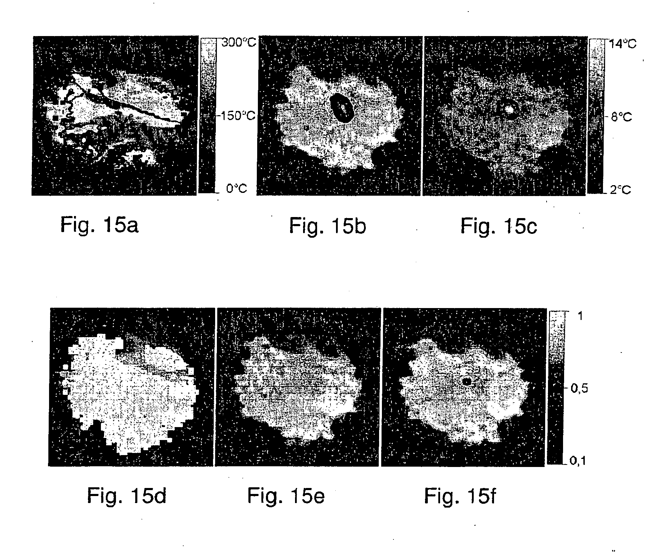

Les figures 15a à 15f permettent de comparer les cartographies de température (figures 15a à 15c) et de dose thermique (figures 15d à 15f), sans correction de la thermométrie et de la position du point de traitement (figures 15a et 15d), avec correction de la thermométrie mais sans correction de la position du point de traitement (figures 15b et 15e), et enfin avec correction de la thermométrie et de la position du point de traitement (figures 15c et 15f).

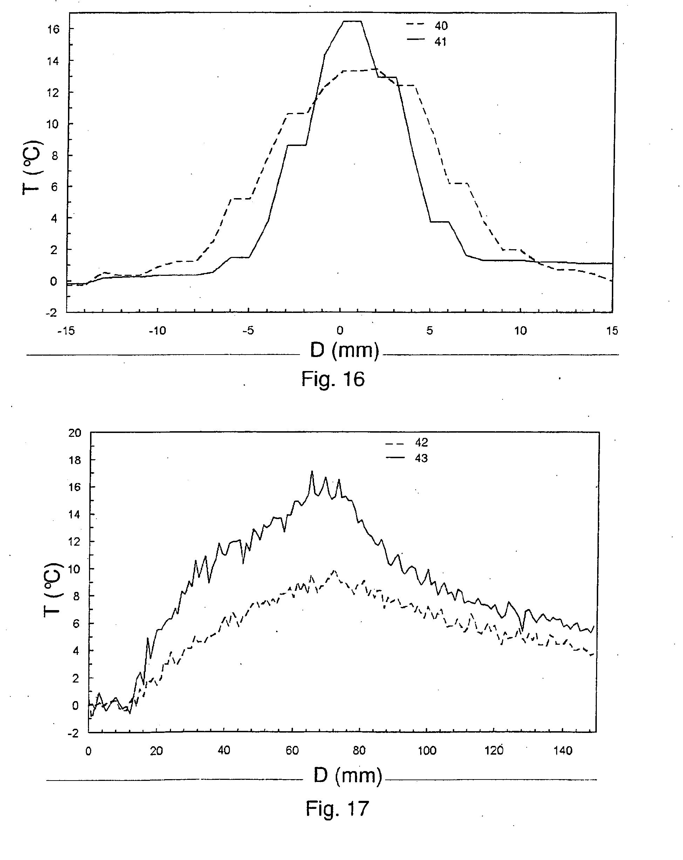

La Figure 16 montre la répartition spatiale de la température le long d'un axe vertical passant par les zones de chauffage présentées sur les cartographies de température, avec (courbe 41) et sans (courbe 40) correction de la position du point de traitement.

La Figure 17 montre l'évolution temporelle de la température dans les zones chauffées au cours de cette expérience avec (courbe 42) et sans (courbe 43) correction de la position du point de traitement.

L'erreur de température commise sans correction de la thermométrie peut atteindre 300°C en 1 minute. Des changements de phase supérieurs à 2Π apparaissent entre deux dynamiques successives, ce qui induit une erreur de température qui s'accumule au cours du temps. Cet artefact masque complètement le chauffage effectué. De même la dose thermique calculée est inexploitable.

Quand aucune correction du point de focalisation n'est effectuée, la zone de chauffage est étalée sur 12mm. Le chauffage induit ne mène pas à une nécrose tissulaire car la montée en température est insuffisante.

Lorsque le déplacement du point de focalisation compense le mouvement de la cible, une zone de chauffage circulaire indique que le mouvement a été corrigé avec justesse. La forme logarithmique continue de la montée en température montre que le chauffage n'est pas affecté par le mouvement. La température atteinte par le tissu est plus élevée que précédemment. L'énergie étant toujours déposée sur la même région tissulaire, une nécrose a été induite.

*Asservissement de la température avec correction de la position du point de traitement*

Le dispositif de traitement thermique avec suivi du déplacement présenté précédemment permet de focaliser à puissance constante toujours au point voulu quelle que soit la position du tissu. La correction de thermométrie donne de plus des cartographies de températures de qualité presque aussi bonnes que celles obtenues sans mouvement. Ces cartes de températures corrigées peuvent servir à effectuer un asservissement de la température comme présenté plus haut. Ainsi les techniques de contrôle de la température ponctuel et spatial ont été appliquées sur un muscle ex vivo soumis un mouvement périodique rigide.

Pour contrôler la température en un point fixe d'un tissu mobile, l'algorithme d'asservissement PID ponctuel de la température détaillé plus haut est effectué avec les cartographies de température corrigées et recalées sur une position centrale du mouvement. Ainsi tous les calculs concernant la puissance nécessaire pour asservir la température sont établis comme si le tissu était immobile. Une fois la puissance requise déterminée la position du point de focalisation est ajustée toutes les 100ms. avec l'algorithme d'anticipation du mouvement périodique décrit précédemment.

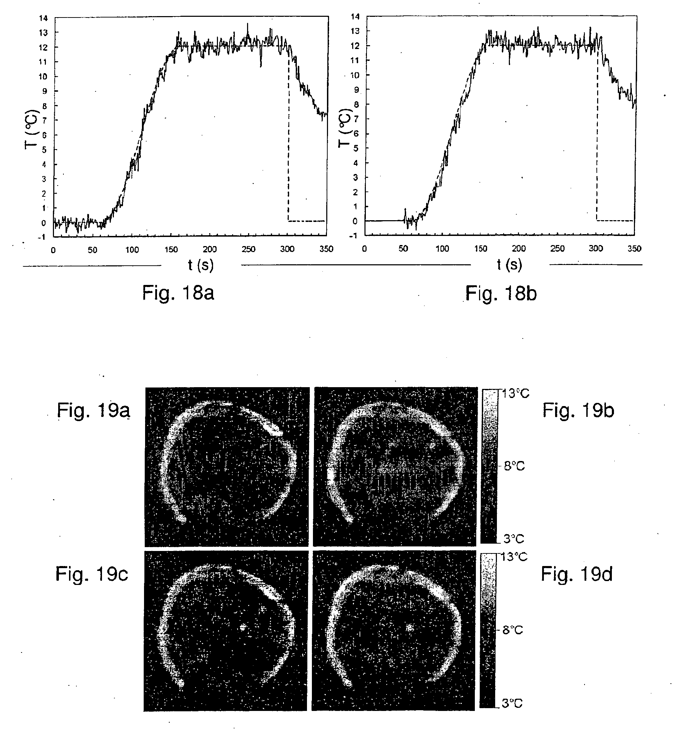

Les figures 18a et 18b permettent de comparer le contrôle de la température effectué sur un muscle ex-vivo immobile (figure 18a) et soumis à un mouvement périodique rigide (figure 18b). Le mouvement de période 6s et d'amplitude 14mm est reconstruit sur une étape de prétraitement de 50 dynamiques. Comme précédemment, chaque dynamique est acquise en 1s avec des voxels de 1,5x1,5x4,5mm$^3$. Le temps de réponse choisi pour effectuer la rétroaction est de 8s. Les paramètres tissulaires utilisés pour anticiper le comportement du tissu par transformée de Fourrier sont de 0,1mm$^2$/s pour le coefficient de diffusion et 0,006K/J pour le coefficient d'absorption.

La consigne de température de 12°C entre 160s et 300s est atteinte avec et sans mouvement du tissu de la même façon avec une précision de 0,45°C. Compte tenu du fait que le bruit de mesure intrinsèque à la séquence utilisée est de 0,3°C, la correction de la thermométrie et les calculs d'asservissement de la température induisent très peu de bruit sur la montée obtenue.

Les figures 19a à 19d représentent des cartographies thermiques correspondant aux dynamiques à 114s (figures 19a et 19b) et à 183s (figures 19c et 19d) de ces deux asservissements ponctuels de la température réalisés sans mouvement (figures 19a et 19c) et avec mouvement (figures 19b et 19d).

Grâce aux techniques de recalage d'images et de correction des artefacts thermométriques, les dynamiques acquises sur le tissu en mouvement sont difficilement différentiables de celles acquises sur le tissu immobile. De plus dans les deux cas, il existe les mêmes isovaleurs de température, comme indiqué par les formes circulaires au tour du point de focalisation. Ceci indique que le point de focalisation est correctement positionné à l'intérieur du tissu quel que soit son déplacement. Dans le cas contraire le chauffage aurait été allongé dans le sens du mouvement (comme à la figure 11 b).

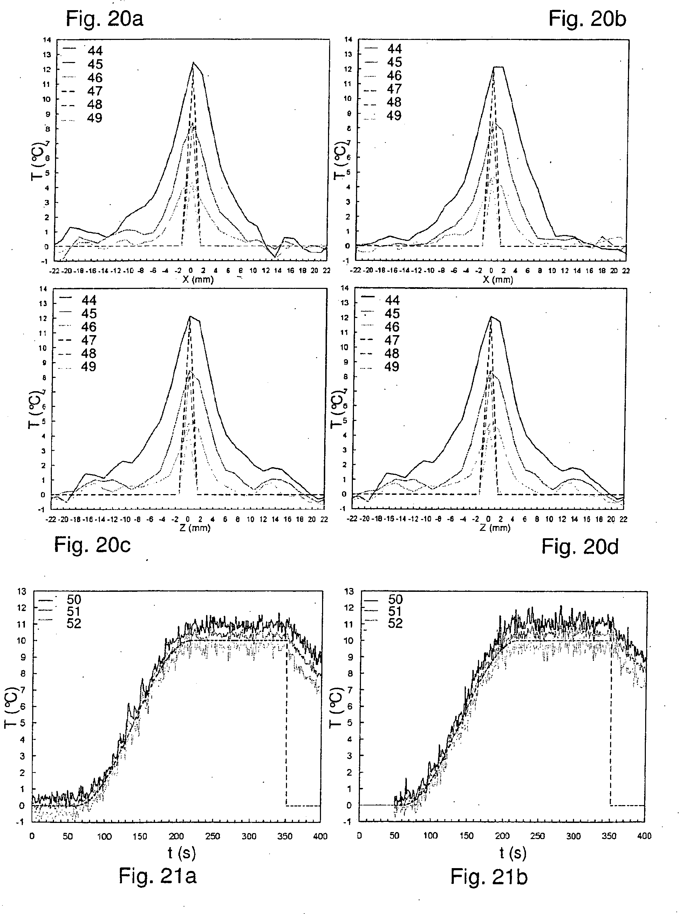

Pour quantifier plus précisément la répartition spatiale de la température entre ces deux expériences, les figures 20a à 20d représentent la température le long des deux axes X et Z sur un muscle ex-vivo immobile (figures 20a en X et 20c en Z) ou soumis à un mouvement périodique rigide (figures 20b en X et 20d en Z), pour les dynamiques 103s (courbes 46), 124s (courbes 45) et 178s (courbes 44).

On constate sur chacun de ces graphes que les températures de consigne (courbes 49 à 103s, courbes 48 à 124s et courbes 47 à 178s) sont précisément respectées. De plus, le point de traitement a été correctement recalé puisque la largeur du chauffage est le même suivant l'axe X et l'axe Z même si un mouvement de 14 mm à lieu le long de l'axe Z.

En revanche en comparant le chauffage sans mouvement avec celui soumis à un mouvement, la répartition spatiale de la température augmente d'environ 10% pour les axes X et Z. Cet élargissement apparaissant simultanément sur les deux axes ne se justifie pas par une imprécision de positionnement du point de traitement mais plutôt par l'addition de lobes secondaires liés au déplacement électronique du point de traitement. Hormis ces lobes secondaires, le contrôle de la température avec suivi du mouvement fonctionne aussi bien sur un tissu mobile qu'immobile.

De la même manière que pour l'asservissement ponctuel de la température, on peut réaliser un asservissement spatial de la température sur un tissu en mouvement. Comme il a été décrit plus haut, on utilise des cartographies de températures corrigées et recalées. La trajectoire fournie par l'algorithme d'asservissement spatial de la température est ensuite modifiée de sorte que les points de focalisation coïncident avec ceux sélectionnés sur l'image recalée. Pour cela chaque point de la trajectoire est subdivisé en une succession de points de durée proche de 100ms. Chacun de ces points est ensuite translaté suivant la valeur de l'anticipation du mouvement calculée comme pour les autres chauffages. De cette façon l'énergie est déposée en chacun des points de comme le définit l'algorithme de contrôle de la température et la position des points focalisation est ajustée selon le mouvement avec un bon échantillonnage temporel.

Les figures 21a et 21b permettent de comparer deux chauffages réalisés avec un asservissement spatial de la température sur un tissu respectivement immobile ou animé d'un mouvement de translation périodique. L'amplitude du mouvement a été réduite à 8mm le long de l'axe Z de sorte que le point de focalisation, puisse être dévié sur une plage de 9mm dans le sens perpendiculaire, l'axe X. De cette façon, même lorsque le tissu se situe en une position extrême du mouvement, le point de focalisation ne s'écarte pas de plus de 6mm de sa position centrale. La largeur des voxels étant de 1,5mm, le contrôle de la température a été effectué sur les 7 voxels centraux de l'axe Z, soit un segment de largeur 9mm. Les courbes 52, 51, et 50, représentent respectivement la valeur de minimum, moyenne et maximale de la température sur ces 7 voxels par rapport à la température de consigne.

La montée en température suit la consigne sur toute la zone de contrôle avec une précision de 0,5°C. La différence entre la température maximum et la température minimum sur les 7 voxels asservis est de 1,2°C sur le tissu immobile et de 1,3°C sur le tissu en mouvement. Avec la technique de suivi du mouvement, le déplacement du tissu n'introduit quasiment aucun bruit sur la mesure thermométrique et sur la précision de contrôle du chauffage.

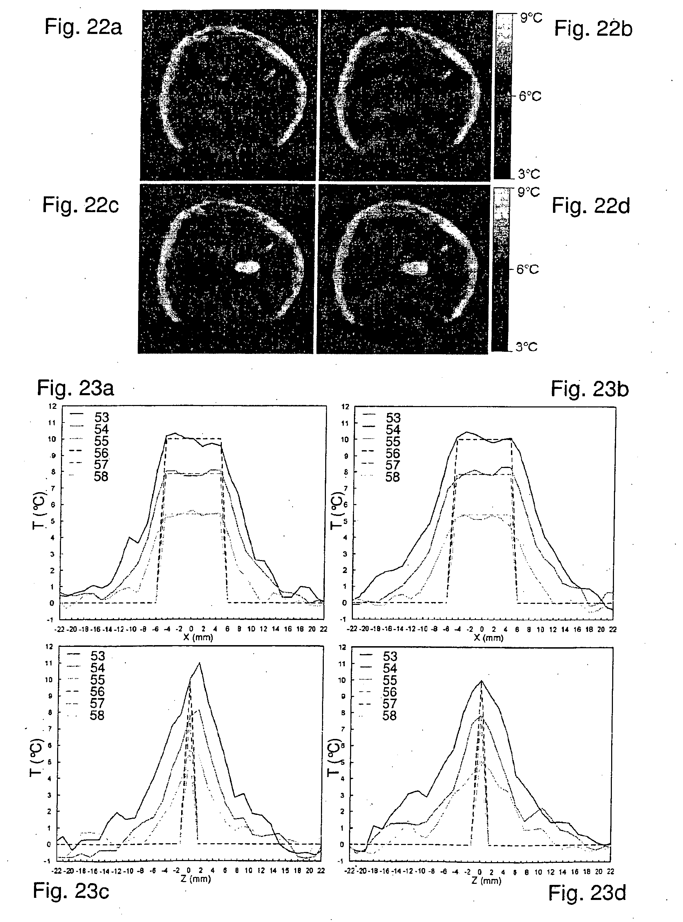

Les figures 22a à 22d représentent des cartographies thermiques correspondant aux dynamiques à 141s (figures 22a et 22b) et à 215s (figures 22c et 22d) de ces deux asservissements ponctuels de la température réalisés sans mouvement (figures 22a et 22c) et avec mouvement (figures 22b et 22d). Le segment allongé de 9mm le long de l'axe X apparaît très distinctement même si le tissu est soumis à un mouvement périodique de 8mm dans une direction perpendiculaire. Puisque le segment chauffé n'est pas déformé, les points de focalisations ont bien suivi le mouvement.

Pour observer plus en détail le contrôle spatial de la température, les figures 23a à 23d représentent la température le long des deux axes X et Z sur un muscle ex-vivo immobile (figures 23a en X et 23c en Z) ou soumis à un mouvement périodique rigide (figures 23b en X et 23d en Z), pour les dynamiques 145s (courbes 55), 172s (courbes 54) et 219s (courbes 53).

La montée en température obtenue correspond au plateau de consigne de température (courbes 58 à 145s, courbes 57 à 172s et courbes 56 à 219s) le long de l'axe X pour chaque dynamique même si l'effet de diffusion thermique s'y oppose, ou que le tissu se déplace dans une direction perpendiculaire l'axe Z. Comme précédemment la répartition spatiale du chauffage sur le tissu en mouvement est légèrement plus large le long des axes X et Z comparativement au chauffage obtenu sur le tissu immobile. Ceci s'explique de même par la présence de lobes d'autant plus importants que le point de focalisation doit s'écarter de sa position centrale.

D'autre part pour le chauffage effectué sur le tissu immobile le point de focalisation est décalé d'un demi-voxel dans le sens de l'axe Z. Ce léger décalage sans graves conséquences arrive fréquemment puisque la résolution spatiale des images IRM utilisée est volontairement proche de la dimension du point de traitement.

Le lecteur aura compris que de nombreuses modifications peuvent être apportées sans sortir matériellement des nouveaux enseignements et des avantages décrits ici. Par conséquent, toutes les modifications de ce type sont destinées à être incorporées à l'intérieur de la portée du dispositif traitement thermique d'un tissu biologique en mouvement selon l'invention, et du procédé de pour le positionnement d'un point (P) dans le traitement thermique <non-invasif> associé.

## Revendications

1. Dispositif de traitement thermique d'une région cible en mouvement d'un tissu biologique, comprenant des moyens générateur d'énergie prévus pour un traitement thermique non-invasif et des moyens de calcul (6) pour estimer une position de la région cible à partir d'un signal de mesure de la région cible,
**caractérisé en ce qu'**il comprend en outre des moyens de commande (7) pour positionner un point de traitement (P) dans la région cible en fonction de la position estimée et d'un délai de positionnement écoulé entre une mesure du signal de mesure de la région cible et le positionnement du point de traitement (P), de façon à compenser le mouvement de la région cible pendant le délai de positionnement.

2. Dispositif selon la revendication 1, **caractérisé en ce que** le délai de positionnement comprend un délai de latence dû à l'estimation de la position de la région cible par les moyens de calcul (6), de sorte que les moyens de commande (7) sont aptes à compenser le mouvement de la région cible pendant le délai de latence.

3. Dispositif selon l'une quelconque des revendications 1 ou 2, **caractérisé en ce que** le délai de positionnement comprend un délai d'anticipation du mouvement de la région cible, de sorte que les moyens de commande (7) sont aptes à anticiper et compenser le mouvement de la région cible pendant le temps d'anticipation.

4. Dispositif selon l'une quelconque des revendications 2 ou 3, **caractérisé en ce qu'**il comprend en outre des moyens de mesure (10) du délai de latence à transmettre aux moyens de commande (7).

5. Dispositif selon l'une quelconque des revendications précédentes, **caractérisé en ce qu'**il comprend en outre des moyens de modélisation pour modéliser le mouvement de la région cible à partir d'une succession de signaux de mesure de la région cible.

6. Dispositif selon la revendication 5, **caractérisé en ce que** la modélisation du mouvement est périodique.

7. Dispositif selon l'une quelconque des revendications 5 ou 6, **caractérisé en ce que** les moyens de modélisation comprennent des moyens pour fournir une position spatiale de la région cible en fonction d'une position temporelle, les positions spatiale et temporelle définissant la position de la région cible.

8. Dispositif selon la revendication 7, **caractérisé en ce que** les moyens de calcul (6) comprennent des moyens pour déterminer une position spatiale estimée de la région cible en utilisant un algorithme de traitement du signal de mesure de la région cible.

9. Dispositif selon la revendication 8, **caractérisé en ce que** les moyens de calcul (6) comprennent des moyens pour déterminer, selon la modélisation du mouvement de la région cible, une position temporelle estimée de la région cible correspondant à la position spatiale estimée.

10. Dispositif selon la revendication 9, **caractérisé en ce que** les moyens de commande (7) comprennent des moyens pour positionner le point de traitement (P) selon une position spatiale réelle de la région cible, la position spatiale réelle étant fonction d'une position temporelle réelle selon la modélisation du mouvement de la région cible, la position temporelle réelle correspondant à la position temporelle estimée augmentée du délai de latence.

11. Dispositif selon la revendication 10, **caractérisé en ce que** les moyens de commande (7) comprennent en outre des moyens pour positionner le point de traitement (P) entre des estimations successives par les moyens de calcul (6) d'une première et d'une deuxième position temporelle estimées à partir respectivement d'un premier et d'un deuxième signal de mesure de la région cible, de façon à anticiper le mouvement de la région cible.

12. Dispositif selon la revendication 11, **caractérisé en ce que** les moyens de commande (7) comprennent des moyens pour positionner le point de traitement (P) selon une position spatiale anticipée, la position spatiale anticipée étant fonction d'une position temporelle anticipée selon la modélisation du mouvement de la région cible, la position temporelle anticipée correspondant à la position temporelle estimée augmentée du délai de latence et du délai d'anticipation.

13. Dispositif selon l'une quelconque des revendications précédentes, **caractérisé en ce que** les moyens de calcul (6) comprennent des moyens pour déterminer un champ de vecteurs de déplacement estimé de la région cible en utilisant un algorithme de traitement du signal de mesure de la région cible.

14. Dispositif selon la revendication 13, **caractérisé en ce que** les moyens de commande (7) comprennent des moyens pour positionner le point de traitement (P) dans la région cible en fonction du champ de vecteurs de déplacement estimé.

**15.** Dispositif selon l'une quelconque des revendications précédentes, **caractérisé en ce qu'**il comprend des moyens d'imagerie (2) comprenant des moyens pour mesurer le signal de mesure de la région cible et des moyens pour fournir une image anatomique de la région cible à partir du signal de mesure de la région cible.

**16.** Dispositif selon la revendication 15, **caractérisé en ce que** les moyens d'imagerie (2) comprennent en outre des moyens pour fournir une image de phase de la région cible à partir du signal de mesure de la région cible, pour le suivi des variations de température de la région cible à partir d'une image de phase de référence.

**17.** Dispositif selon la revendication 16, **caractérisé en ce que** les moyens de calcul (6) comprennent en outre des moyens pour modifier l'image de phase de référence pour corriger une perturbation de température due à un mouvement de la région cible.

**18.** Dispositif selon la revendication 17, **caractérisé en ce que** les moyens de calcul (6) comprennent des moyens pour modifier l'image de phase de référence en fonction d'un historique d'images de phase.

**19.** Dispositif selon l'une quelconque des revendications 15 à 18, **caractérisé en ce qu'**il comprend en outre des moyens de régulation d'un rayonnement appliqué sur le point de traitement (P) dans la région cible pour que la distribution spatiale de température de la région cible soit conforme à une consigne de distribution spatiale de température.

**20.** Dispositif selon la revendication 19, **caractérisé en ce que** les moyens de régulation comprennent des moyens pour réguler le rayonnement appliqué en fonction de la distribution spatiale de température de la région cible et d'une consigne de distribution spatiale de température, selon une loi de régulation comprenant un terme Proportionnel-Intégral-Dérivé.

**21.** Procédé de positionnement d'un point (P) dans le traitement thermique non-invasif d'une région cible en mouvement d'un tissu biologique, comprenant les étapes consistant à :

- mesurer la région cible pour obtenir un signal de mesure de la région cible,
- estimer une position de la région cible à partir du signal de mesure de la région cible par des moyens de calcul (6),

-**caractérisé en ce qu'**il comprend une étape consistant à positionner un point de traitement (P) dans la région cible par des moyens de commande (7), ledit positionnement étant fonction de la position estimée et d'un délai de positionnement écoulé entre la mesure du signal de mesure de la région cible et le positionnement du point de traitement, de façon à compenser le mouvement de la région cible pendant le délai de positionnement.

**22.** Procédé selon la revendication 21, **caractérisé en ce que** le délai de positionnement comprend un délai de latence dû à l'estimation de la position de la région cible par les moyens de calcul (6), de façon à compenser le mouvement de la région cible pendant le délai de latence.

**23.** Procédé selon l'une quelconque des revendications 21 ou 22, **caractérisé en ce que** le délai de positionnement comprend un délai d'anticipation du mouvement de la région cible, de façon à anticiper et compenser le mouvement de la région cible pendant le temps d'anticipation.

**24.** Procédé selon l'une quelconque des revendications 22 ou 23, **caractérisé en ce qu'**il comprend en outre l'étape consistant à mesurer en temps réel le délai de latence par des moyens de mesure (10).

**25.** Procédé selon l'une quelconque des revendications 21 à 24, **caractérisé en ce qu'**il comprend en outre l'étape consistant à modéliser le mouvement de la région cible à partir d'une succession de signaux de mesure de la région cible.

**26.** Procédé selon la revendication 25, **caractérisé en ce que** l'on détermine une période type pour la modélisation du mouvement de la région cible.

**27.** Procédé selon l'une quelconque des revendications 25 ou 26, **caractérisé en ce que** l'on modélise la position de la région cible par une position spatiale en fonction d'une position temporelle.

**28.** Procédé selon la revendication 27, **caractérisé en ce que** l'estimation de la position de la région cible comprend les étapes consistant à :

- déterminer une position spatiale estimée de la région cible en utilisant un algorithme de traitement du signal de mesure de la région cible, et
- déterminer, selon la modélisation du mouvement de la région cible, une position temporelle estimée de la région cible correspondant à la position spatiale estimée.

**29.** Procédé selon la revendication 28, **caractérisé en ce que** le positionnement du point de traitement (P) comprend les étapes consistant à :

- déterminer une position temporelle réelle de la région cible, la position temporelle réelle correspondant à la position temporelle estimée augmentée du délai de latence,
- déterminer une position spatiale réelle de la région cible, selon la modélisation du mouvement de la région cible, en fonction de la position temporelle réelle, et
- positionner le point de traitement (P) selon la position spatiale réelle.

**30.** Procédé selon la revendication 29, **caractérisé en ce que** le positionnement du point de traitement (P) comprend les étapes supplémentaires consistant à :

- déterminer une position temporelle anticipée de la région cible, la position temporelle anticipée correspondant à la position temporelle estimée augmentée du délai de latence et d'un délai d'anticipation,
- déterminer une position spatiale anticipée de la région cible, selon la modélisation du mouvement de la région cible, en fonction de la position temporelle anticipée, et
- positionner le point de traitement (P) selon la position spatiale anticipée.

**31.** Procédé selon la revendication 30, **caractérisé en ce que** les étapes supplémentaires du positionnement du point de traitement (P) sont renouvelées jusqu'à ce qu'une nouvelle position temporelle estimée soit déterminée.

**32.** Procédé selon l'une quelconque des revendications 23 à 31, **caractérisé en ce qu'**il comprend en outre l'étape consistant à déterminer un champ de vecteurs de déplacement estimé de la région cible en utilisant un algorithme de traitement du signal de mesure de la région cible.

**33.** Procédé selon la revendication 32, **caractérisé en ce que** l'on positionne le point de traitement dans la région cible en fonction du champ de vecteurs de déplacement estimé.

**34.** Procédé selon l'une quelconque des revendications 23 à 33, **caractérisé en ce que** le signal de mesure de la région cible fournit une image anatomique de la région cible pour le positionnement du point de traitement.

**35.** Procédé selon la revendication 34, **caractérisé en ce que** le signal de mesure de la région cible fournit en outre une image de phase de la région cible.

**36.** Procédé selon la revendication 35, **caractérisé en ce qu'**il comprend en outre les étapes consistant à :

- déterminer une image de phase de référence ;
- comparer l'image de phase acquise et l'image de phase de référence pour suivre les variations de température de la région cible.

**37.** Procédé selon la revendication 36, **caractérisé en ce que** l'on détermine une image de phase de référence à partir d'un historique d'images de phase.

**38.** Procédé selon la revendication 37, **caractérisé en ce qu'**il comprend en outre une étape consistant à réguler un rayonnement appliqué sur le point de traitement (P) dans la région cible pour que la distribution spatiale de température de la région cible soit conforme à une consigne de distribution spatiale de température.

**39.** Procédé selon la revendication 38, **caractérisé en ce que** l'on régule le rayonnement appliqué en fonction de la distribution spatiale de température de la région cible et d'une consigne de distribution spatiale de température, selon une loi de régulation comprenant un terme Proportionnel-Intégral-Dérivé.

**Claims**

1. A heat treatment device of a target area in motion of biological tissue, comprising energy generating means provided for a non-invasive heat treatment and calculation means (6) for estimating a position of the target area using a measurement signal of the target area, **characterized in that** it further comprises controlling means (7) for positioning a treatment focal point (P) in the target area based on the estimated position and a positioning time lag between a measurement of the measurement signal of the target area and the positioning of the treatment focal point (P), so as to compensate the movement of the target area during the positioning time lag.

2. The device of claim 1, **characterized in that** the positioning time lag comprises a latency time due to the estimation of the position of the target area using the calculation means (6), so that the control means (7) are adapted for compensating the movement of the target area during the latency time.

3. The device of any of claims 1 or 2, **characterized in that** the positioning time lag comprises a prediction time of the movement of the target area, so that the control means (7) are adapted for predicting and compensating the movement of the target area during the prediction time.

4. The device of any of claims 2 or 3, **characterized in that** it further comprises measurement means (10) of the latency time to transmit to the control means (7).

5. The device of any of the preceding claims, **characterized in that** it further comprises modeling means for modeling the movement of the target area based on a series of measurement signals of the target area.

6. The device of claim 5, **characterized in that** the modeling of the movement is periodic.

7. The device of any of claims 5 or 6, **characterized in that** the modeling means comprise means to provide a spatial position of the target area based on a temporal position, with the spatial and temporal positions defining the position of the target area.

8. The device of claim 7, **characterized in that** the calculation means (6) comprise means to determine an estimated spatial position of the target area using an algorithm for processing of the measurement signal of the target area.

9. The device of claim 8, **characterized in that** the calculation means (6) comprise means to determine, according to the modeling of the movement of the target area, an estimated temporal position of the target area corresponding to the estimated spatial position.

10. The device of claim 9, **characterized in that** the control means (7) comprise means to position the treatment focal point (P) in accordance with a real temporal position of the target area, the real spatial position being based on the real temporal position according to the modeling of the movement of the target area, the real temporal position corresponding to the estimated temporal position enhanced by the latency time.

11. The device of claim 10, **characterized in that** the control means (7) further comprise means to position the treatment focal point (P) between successive estimations performed by the calculation means (6) of a first and second temporal position estimated respectively from a first and second measurement signal of the target area, in order to predict the movement of the target area.

12. The device of claim 11, **characterized in that** the control means (7) comprise means to position the treatment focal point (P) according to a predicted spatial position, the predicted spatial position being a factor of a predicted temporal position according to the modeling of the movement of the target area, the predicted temporal position corresponding to the estimated temporal position enhanced by the latency time and the prediction time.

13. The device of any of the preceding claims, **characterized in that** the calculation means (6) comprise means to determine an estimated displacement vector field of the target area using an algorithm for processing of the measurement signal of the target area.

14. The device of claim 13, **characterized in that** the control means (7) comprise means to position the treatment focal point (P) in the target area as a factor of the estimated displacement vector field.

15. The device of any of the preceding claims, **characterized in that** it comprises imaging means (2) comprising means to measure the measurement signal of the target area and means to provide an anatomic image of the target area using the measurement signal of the target area.

16. The device of claim 15, **characterized in that** the imaging means (2) further comprise means to provide a phase image of the target area using the measurement signal of the target area, for monitoring temperature variations of the target area using a reference phase image.

17. The device of claim 16, **characterized in that** the calculation means (6) further comprise means to modify the reference phase image to correct a disturbance of the temperature due to a movement of the target area.

18. The device of claim 17, **characterized in that** the calculation means (6) comprise means to modify the reference phase image using stored phase images.

19. The device of any of claims 15 to 18, **characterized in that** it further comprises regulation means of a radiation applied on the treatment focal point (P) in the target area so that the spatial distribution of temperature of the target area should conform with a setting for the spatial distribution of temperature.

20. The device of claim 19, **characterized in that** the regulation means comprise means to regulate the applied radiation as a factor of the spatial distribution of temperature in the target area and a setting for the spatial distribution of temperature, in accordance with a regulation equation comprising a Proportional-Integral-Derived term.

21. A method of positioning a focal point (P) in the non-invasive heat treatment of a target area in motion of a biological tissue, comprising the steps consisting of:

    - Measuring the target area for obtaining a measurement signal of the target area,
    - Estimating a position of the target area based on the measurement signal of the target area using calculation means (6),
    - **characterized in that** it comprises a step consisting of positioning a treatment focal point (P) in the target area using control means (7), said positioning being dependent on the estimated position and a positioning time lag between the measurement of the measurement signal of the target area and the positioning of the treatment focal point, so as to compensate the movement of the target area during the positioning time lag.

22. The method of claim 21, **characterized in that** the positioning time lag comprises a latency time due to the estimation of the position of the target area using the calculation means (6), so as to compensate the movement of the target area during the latency time.

23. The method of any of claims 21 or 22, **characterized in that** the positioning time lag comprises a prediction time of the movement of the target area, so as to predict and compensate the movement of the target area during the prediction time.

24. The method of any of claims 22 or 23, **characterized in that** it further comprises the step of measuring the latency time in real time using measurement means (10).

25. The method of any of claims 21 to 24, **characterized in that** it further comprises the step of modeling the movement of the target area based on a series of measurement signals of the target area.

26. The method of claim 25, **characterized in that** a period for modeling the movement of the target area is determined.

27. The method of any of claims 25 or 26, **characterized in that** the position of the target area is modeled using a spatial position as a factor of a temporal position.

28. The method of claim 27, **characterized in that** the estimation of the position of the target area comprises the steps of:

    - Determining the estimated spatial position of the target area using an algorithm for processing the measurement signal of the target area, and
    - Determining, according to the modeling of the movement of the target area, an estimated temporal position corresponding to the estimated spatial position.

29. The method of claim 28, **characterized in that** the positioning of the treatment focal point (P) comprises the steps of:

- Determining a real temporal position of the target area, the real temporal position corresponding to the estimated temporal position enhanced by the latency time,
- Determining a real temporal position of the target area, according to the modeling of the movement of the target area, based on the real temporal position , and
- Positioning the treatment focal point (P) according to the real spatial position.

30. The method of claim 29, **characterized in that** the positioning of the treatment focal point (P) comprises the additional steps of:

- Determining a predicted temporal position of the target area, the predicted temporal position corresponding to the estimated temporal position enhanced by the latency time and a prediction time,
- Determining a predicted spatial position of the target area, according to the modeling of the movement of the target area, based on the predicted temporal position, and
- Positioning the treatment focal point (P) based on the predicted spatial position.

31. The method of claim 30, **characterized in that** the additional steps for positioning the treatment focal point (P) are repeated until a new estimated temporal position is determined.

32. The method of any of claims 23 to 31, **characterized in that** it further comprises the step of determining an estimated displacement vector field of the target area using an algorithm for processing the measurement signal of the target area.

33. The method of claim 32, **characterized in that** the treatment focal point is positioned in the target area based on the estimated displacement vector field.

34. The method of any of claims 23 to 33, **characterized in that** the measurement signal of the target area provides an anatomic image of the target area for positioning the treatment focal point.

35. The method of claim 34, **characterized in that** the measurement signal of the target area further provides a phase image of the target area.

36. The method of claim 35, **characterized in that** it further comprises the steps of:

- Determining a reference phase image;
- Comparing the acquired phase image and the reference phase image for monitoring the temperature variations in the target area.

37. The method of claim 36, **characterized in that** a reference phase image is determined from the stored phase images.

38. The method of claim 37, **characterized in that** it further comprises the step of regulating the radiation applied to the treatment focal point (P) in the target area so that the spatial distribution of temperature in the target area conforms with a setting for the spatial distribution of temperature.

39. The method of claim 38, **characterized in that** the radiation applied is regulated based on the spatial distribution of the temperature in the target area and the setting for the spatial distribution of temperature, according to a regulation equation comprising a Proportional-Integral-Derived term.

**Patentansprüche**

1. Vorrichtung zur thermischen Behandlung einer beweglichen Zielregion eines biologischen Gewebes, umfassend Mittel zur Energieerzeugung, die für eine nicht-invasive Wärmebehandlung bestimmt sind, und Kalkulationsmittel (6) zum Abschätzen einer Position der Zielregion ausgehend von einem Messsignal der Zielregion,
**dadurch gekennzeichnet, dass** die Vorrichtung außerdem Steuerungsmittel (7) umfasst, die einen Behandlungspunkt (P) in der Zielregion in Abhängigkeit von der geschätzten Position und einer Positionierungs-Verzögerungszeit, die zwischen einem Messen des Messsignals der Zielregion und der Positionierung des Behandlungspunkts (P) vergeht, derart positionieren, dass die Bewegung der Zielregion während der Positionierungs-Verzögerungszeit kompensiert wird.

2. Vorrichtung gemäß Anspruch 1, **dadurch gekennzeichnet, dass** die Positionierungs-Verzögerungszeit eine Latenzzeit umfasst, die auf die Abschätzung der Position der Zielregion durch die Kalkulationsmittel (6) zurückzuführen ist, so dass die Steuerungsmittel (7) in der Lage sind, die Bewegung der Zielregion während der Latenzzeit zu kompensieren.

3. Vorrichtung gemäß irgendeinem der Ansprüche 1 oder 2, **dadurch gekennzeichnet, dass** die Positionierungs-Verzögerungszeit eine Antizipationszeit der Bewegung der Zielregion umfasst, so dass die Steuerungsmittel (7) in der Lage sind, die Bewegung der Zielregion während der Antizipationszeit zu antizipieren und zu kompensieren.

4. Vorrichtung gemäß irgendeinem der Ansprüche 2 oder 3, **dadurch gekennzeichnet, dass** sie außerdem Mittel (10) zum Messen der Latenzzeit, die an die Steuerungsmittel (7) übermittelt wird, umfasst.

5. Vorrichtung gemäß irgendeinem der vorhergehenden Ansprüche, **dadurch gekennzeichnet, dass** sie außerdem Modellierungsmittel zur Darstellung der Bewegung der Zielregion im Modell, ausgehend von einer Aufeinanderfolge von Messsignalen der Zielregion, umfasst.

6. Vorrichtung gemäß Anspruch 5, **dadurch gekennzeichnet, dass** die Darstellung der Bewegung im Modell periodisch ist.

7. Vorrichtung gemäß irgendeinem der Ansprüche 5 oder 6, **dadurch gekennzeichnet, dass** die Modellierungsmittel Mittel zur Lieferung einer Raumposition der Zielregion in Abhängigkeit von einer Zeitposition umfassen, wobei die Raum- und Zeitpositionen die Position der Zielregion definieren.

8. Vorrichtung gemäß Anspruch 7, **dadurch gekennzeichnet, dass** die Kalkulationsmittel (6) Mittel zur Bestimmung einer geschätzten Raumposition der Zielregion unter Verwendung eines Algorithmus zur Verarbeitung des Messsignals der Zielregion umfassen.

9. Vorrichtung gemäß Anspruch 8, **dadurch gekennzeichnet, dass** die Kalkulationsmittel (6) Mittel umfassen, die, entsprechend der Darstellung der Bewegung der Zielregion im Modell, die Bestimmung einer geschätzten Zeitposition der Zielregion, die der geschätzten Raumposition entspricht, durchführen.

10. Vorrichtung gemäß Anspruch 9, **dadurch gekennzeichnet, dass** die Steuerungsmittel (7) Mittel zur Positionierung des Behandlungspunkts (P) entsprechend einer tatsächlichen Raumposition der Zielregion umfassen, wobei die tatsächliche Raumposition von einer tatsächlichen Zeitposition, entsprechend der Darstellung der Bewegung der Zielregion im Modell, abhängig ist, wobei die tatsächliche Zeitposition der geschätzten, um die Latenzzeit erhöhten Zeitposition entspricht.

11. Vorrichtung gemäß Anspruch 10, **dadurch gekennzeichnet, dass** die Steuerungsmittel (7) außerdem Mittel zur Positionierung des Behandlungspunkts (P) zwischen den durch die Kalkulationsmittel (6) durchgeführten, aufeinander folgenden Abschätzungen einer ersten und einer zweiten Zeitposition, die jeweils ausgehend von einem ersten und einem zweiten Messsignal der Zielregion abgeschätzt wurden, umfassen, derart, das die Bewegung der Zielregion antizipiert wird.

12. Vorrichtung gemäß Anspruch 11, **dadurch gekennzeichnet, dass** die Steuerungsmittel (7) Mittel zur Positionierung des Behandlungspunkts (P) gemäß einer antizipierten Raumposition umfassen, wobei die antizipierte Raumposition von einer antizipierten Zeitposition, entsprechend der Darstellung der Bewegung der Zielregion im Modell, abhängig ist, wobei die antizipierte Zeitposition der geschätzten, um die Latenzzeit und die Antizipationszeit erhöhten Zeitposition entspricht.

13. Vorrichtung gemäß irgendeinem der vorhergehenden Ansprüche, **dadurch gekennzeichnet, dass** die Kalkulationsmittel (6) Mittel zur Bestimmung eines geschätzten Verschiebungsvektorfelds der Zielregion unter Verwendung eines Algorithmus zur Verarbeitung des Messsignals der Zielregion umfassen.

14. Vorrichtung gemäß Anspruch 13, **dadurch gekennzeichnet, dass** die Steuerungsmittel (7) Mittel zur Positionierung des Behandlungspunkts (P) in der Zielregion in Abhängigkeit von dem geschätzten Verschiebungsvektorfeld umfassen.

**15.** Vorrichtung gemäß irgendeinem der vorhergehenden Ansprüche, **dadurch gekennzeichnet, dass** sie Mittel zur Bilddarstellung (2) umfasst, welche Mittel zum Messen des Messsignals der Zielregion und Mittel zur Lieferung eines anatomischen Bilds der Zielregion, ausgehend von dem Messsignal der Zielregion, umfassen.

**16.** Vorrichtung gemäß Anspruch 15, **dadurch gekennzeichnet, dass** die Bilddarstellungsmittel (2) außerdem Mittel zur Lieferung eines Phasenbilds der Zielregion, ausgehend von dem Messsignal der Zielregion, umfassen, um Temperaturänderungen in der Zielregion ausgehend von einem Vergleichsphasenbild zu verfolgen.

**17.** Vorrichtung gemäß Anspruch 16, **dadurch gekennzeichnet, dass** die Kalkulationsmittel (6) außerdem Mittel zur Modifizierung des Vergleichsphasenbilds umfassen, um eine Temperaturstörung, die durch eine Bewegung der Zielregion hervorgerufen wird, zu korrigieren.

**18.** Vorrichtung gemäß Anspruch 17, **dadurch gekennzeichnet, dass** die Kalkulationsmittel (6) Mittel zur Modifizierung des Vergleichsphasenbilds in Abhängigkeit einer Historie von Phasenbildern umfassen.

**19.** Vorrichtung gemäß irgendeinem der Ansprüche 15 bis 18, **dadurch gekennzeichnet, dass** sie außerdem Mittel zur Regulation einer Strahlung, die auf den Behandlungspunkt (P) in der Zielregion angewendet wird, umfasst, so dass die zeitliche Temperaturverteilung der Zielregion mit einer Vorgabe der räumlichen Temperaturverteilung übereinstimmt.

**20.** Vorrichtung gemäß Anspruch 19, **dadurch gekennzeichnet, dass** die Regulationsmittel Mittel zur Regulierung der angewendeten Strahlung in Abhängigkeit von der räumlichen Temperaturverteilung in der Zielregion und einer Vorgabe der räumlichen Temperaturverteilung gemäß einem Regulationsgesetz, welches einen Proportional-Integral-Differential-Term umfasst, umfassen.

**21.** Verfahren zur Positionierung eines Punkts (P) bei der thermischen, nicht-invasiven Behandlung einer beweglichen Zielregion eines biologischen Gewebes, umfassend die folgenden Schritte:

    - Messen der Zielregion zum Erhalt eines Messsignals der Zielregion,
    - Abschätzen einer Position der Zielregion, ausgehend von dem Messsignal der Zielregion, durch Kalkulationsmittel (6),

**dadurch gekennzeichnet, dass** das Verfahren einen Schritt beinhaltet, in dem ein Behandlungspunkt (P) in der Zielregion durch Steuerungsmittel (7) positioniert wird, wobei die Positionierung abhängig ist von der geschätzten Position und von einer Positionierungs-Verzögerungszeit, die zwischen dem Messen des Messsignals der Zielregion und der Positionierung des Behandlungspunkts (P) vergeht, so dass die Bewegung der Zielregion während der Positionierungs-Verzögerungszeit kompensiert wird.

**22.** Verfahren gemäß Anspruch 21, **dadurch gekennzeichnet, dass** die Positionierungs-Verzögerungszeit eine Latenzzeit umfasst, die auf die Abschätzung der Position der Zielregion durch die Kalkulationsmittel (6) zurückzuführen ist, so dass die Bewegung der Zielregion während der Latenzzeit kompensiert wird.

**23.** Verfahren gemäß irgendeinem der Ansprüche 21 oder 22, **dadurch gekennzeichnet, dass** die Positionierungs-Verzögerungszeit eine Antizipationszeit der Bewegung der Zielregion umfasst, so dass die Bewegung der Zielregion während der Antizipationszeit antizipiert und kompensiert wird.

**24.** Verfahren gemäß irgendeinem der Ansprüche 22 oder 23, **dadurch gekennzeichnet, dass** es außerdem einen Schritt umfasst, in dem in Echtzeit die Latenzzeit durch die Messmittel (10) gemessen wird.

**25.** Verfahren gemäß irgendeinem der Ansprüche 21 bis 24, **dadurch gekennzeichnet, dass** es außerdem einen Schritt umfasst, in dem die Bewegung der Zielregion, ausgehend von einer Aufeinanderfolge von Messsignalen der Zielregion, im Modell dargestellt wird.

**26.** Verfahren gemäß Anspruch 25, **dadurch gekennzeichnet, dass** ein Zeitraum-Typ für die Darstellung der Bewegung der Zielregion im Modell bestimmt wird.

**27.** Verfahren gemäß irgendeinem der Ansprüche 25 oder 26, **dadurch gekennzeichnet, dass** die Position der Zielregion durch eine Raumposition in Abhängigkeit von einer Zeitposition im Modell dargestellt wird.

**28.** Verfahren gemäß Anspruch 27, **dadurch gekennzeichnet, dass** die Abschätzung der Position der Zielregion folgende Schritte umfasst:

- Bestimmung einer geschätzten Raumposition der Zielregion unter Verwendung eines Algorithmus zur Verarbeitung des Messsignals der Zielregion, und
- Bestimmung, entsprechend der Darstellung der Bewegung der Zielregion im Modell, einer geschätzten Zeitposition der Zielregion, die der geschätzten Raumposition entspricht.

**29.** Verfahren gemäß Anspruch 28, **dadurch gekennzeichnet, dass** die Positionierung des Behandlungspunkts (P) folgende Schritte umfasst:

- Bestimmung einer tatsächlichen Zeitposition der Zielregion, wobei die tatsächliche Zeitposition der geschätzten, um die Latenzzeit erhöhten Zeitposition entspricht,
- Bestimmung einer tatsächlichen Raumposition der Zielregion entsprechend der Darstellung der Bewegung der Zielregion im Modell, in Abhängigkeit von der tatsächlichen Zeitposition, und
- Positionieren des Behandlungspunkts (P) entsprechend der tatsächlichen Raumposition.

**30.** Verfahren gemäß Anspruch 29, **dadurch gekennzeichnet, dass** die Positionierung des Behandlungspunkts (P) folgende zusätzliche Schritte umfasst:

- Bestimmung einer antizipierten Zeitposition der Zielregion, wobei die antizipierte Zeitposition der geschätzten, um die Latenzzeit und die Antizipationszeit erhöhten Zeitposition entspricht,
- Bestimmung einer antizipierten Raumposition der Zielregion, entsprechend der Darstellung der Bewegung der Zielregion im Modell, in Abhängigkeit von der antizipierten Zeitposition, und
- Positionieren des Behandlungspunkts (P) entsprechend der antizipierten Raumposition.

**31.** Verfahren gemäß Anspruch 30, **dadurch gekennzeichnet, dass** die zusätzlichen Schritte der Positionierung des Behandlungspunkts (P) solange wiederholt werden, bis eine neue geschätzte Zeitposition bestimmt wurde.

**32.** Verfahren gemäß irgendeinem der Ansprüche 23 bis 31, **dadurch gekennzeichnet, dass** es außerdem einen Schritt beinhaltet, in dem ein geschätztes Verschiebungsvektorfeld der Zielregion unter Verwendung eines Algorithmus zur Verarbeitung des Messsignals der Zielregion bestimmt wird.

**33.** Verfahren gemäß Anspruch 32, **dadurch gekennzeichnet, dass** der Behandlungspunkt in der Zielregion in Abhängigkeit von dem geschätzten Verschiebungsvektorfeld positioniert wird.

**34.** Verfahren gemäß irgendeinem der Ansprüche 23 bis 33, **dadurch gekennzeichnet, dass** das Messsignal der Zielregion ein anatomisches Bild der Zielregion für die Positionierung des Behandlungspunkts liefert.

**35.** Verfahren gemäß Anspruch 34, **dadurch gekennzeichnet, dass** das Messsignal der Zielregion außerdem ein Phasenbild der Zielregion liefert.

**36.** Verfahren gemäß Anspruch 35, **dadurch gekennzeichnet, dass** es außerdem die folgenden Schritte umfasst:

- Bestimmung eines Vergleichsphasenbilds,
- Vergleichen des erhaltenen Phasenbilds mit dem Vergleichsphasenbild zum Verfolgen von Temperaturänderungen der Zielregion.

**37.** Verfahren gemäß Anspruch 36, **dadurch gekennzeichnet, dass** ein Vergleichsphasenbild ausgehend von einer Historie von Phasenbildern bestimmt wird.

**38.** Verfahren gemäß Anspruch 37, **dadurch gekennzeichnet, dass** es außerdem einen Schritt umfasst, in dem eine Strahlung, die auf den Behandlungspunkt (P) in der Zielregion angewendet wird, reguliert wird, um die räumliche Temperaturverteilung der Zielregion mit einer Vorgabe der räumlichen Temperaturverteilung in Übereinstimmung zu bringen.

**39.** Verfahren gemäß Anspruch 38, **dadurch gekennzeichnet, dass** die angewendete Strahlung in Abhängigkeit von der räumlichen Temperaturverteilung der Zielregion und einer Vorgabe der räumlichen Temperaturverteilung gemäß einem Regulationsgesetz, welches einen Proportional-Integral-Differential-Term umfasst, reguliert wird.

Fig. 1

Fig. 2a

Fig. 2b

Fig. 2c

Fig. 2d

Fig. 2e

13

16    14    11

17

15

18

12

Fig. 3

Fig. 4a

Fig. 4b

Fig. 5a

Fig. 5b

Fig. 6a

Fig. 6b

Fig. 7a

Fig. 7b

Fig. 8a          Fig. 8b.          Fig. 8c

Fig. 8d          Fig. 8e          Fig. 8f

Fig. 9

Fig. 10

Fig. 11a  Fig. 11b  Fig. 11c

Fig. 11d  Fig. 11e  Fig. 11f

Fig. 12

Fig. 13

Fig. 14a

Fig. 14b

Fig. 15a          Fig. 15b          Fig. 15c

Fig. 15d          Fig. 15e          Fig. 15f

Fig. 16

Fig. 17

Fig. 18a

Fig. 18b

Fig. 19a

Fig. 19b

Fig. 19c

Fig. 19d

Fig. 20a

Fig. 20b

Fig. 20c

Fig. 20d

Fig. 21a

Fig. 21b

Fig. 22a

Fig. 22b

Fig. 22c

Fig. 22d

Fig. 23a

Fig. 23b

Fig. 23c

Fig. 23d

## RÉFÉRENCES CITÉES DANS LA DESCRIPTION

**Documents brevets cités dans la description**

- EP 1110508 A
- US 5938600 A